# EUROPEAN PATENT APPLICATION

(11) **EP 4 050 106 A1**
(43) Date of publication of application: **31.08.2022**
(21) Application number: 20879390.1
(22) Date of filing: 23.10.2020
(51) Int. Cl.: C12P 19/04, C12N 1/21, C12N 15/54

(54) **METABOLIZING-ENZYME-DESTROYED STRAIN OF AEROBIC BACTERIUM, AND METHOD FOR CULTURING SAME**

(30) Priority: 25.10.2019 JP 2019194513
(71) Applicant: Nagase & Co., Ltd., Osaka 550-8668 (JP)
(72) Inventor: YAMAMOTO, Shogo, Kobe-shi, Hyogo 651-2241 (JP)
(74) Representative: Marks & Clerk LLP
(86) International application number: PCT/JP2020/039840
(87) International publication number: WO 2021/079972

(57) **Abstract**

The invention relates to a metabolic enzyme-disrupted aerobic strain and a method for culturing the strain. The present invention provides, for example, a culture comprising a culture medium that has been cultured under an aerobic condition, wherein the culture medium contains an aerobe, wherein the aerobe has a disrupted gene encoding a metabolic enzyme of glycolysis selected from the group consisting of the metabolic enzymes of glycolysis except hexokinase, thereby suppressing metabolism from a carbon source (e.g., glucose) to the TCA cycle in the aerobe.

## Description

### Technical Field

The present invention relates to a metabolic enzyme-disrupted aerobic strain and a method for culturing the strain.

### Background Art

Microbial secondary metabolites include many industrially useful substances. For substance production, microorganisms are used. Aerobe utilize carbon sources for proliferation and produce secondary metabolites under an aerobic condition. The yield of a secondary metabolite is defined relative to the amount of a carbon source (for example, glucose) added to a culture medium and referred to as a "yield relative to sugar". In microbial substance production, improving the yield of a substance relative to sugar is a goal.

Of the aerobe, actinomycetes are known as the aerobe producing useful secondary metabolites such as antibiotics. However, the production amounts and "yields relative to sugar" of the secondary metabolites are low. Most secondary metabolites are limitedly used in industry (for example, used in pharmaceuticals). Attempts have been made to increase the production amount of secondary metabolites by disrupting enzymes in metabolic pathways (Patent Literature 1). However, disruption of an enzyme is a methodology to a specific metabolite and lacks versatility.

### Citation List

### Patent Literature

Patent Literature 1: WO2017/150304

### Summary of Invention

The present invention provides a metabolic enzyme-disrupted aerobic strain and a method for culturing the strain.

The present inventors disrupted a gene encoding a metabolic enzyme selected from the group consisting of the metabolic enzymes of glycolysis except hexokinase to inhibit use of a carbon source such as glucose for cell proliferation and to facilitate use of a carbon source in substance production; at the same time, they supplied a metabolite of glycolysis in the downstream of the metabolic enzyme and/or a metabolite of the metabolic pathway flowing into the downstream thereof to cells, with the result that a method for proliferating the cells was found.

According to the present invention, for example, the following inventions are provided.
[1] A culture including a culture medium used in culturing under an aerobic condition, wherein
   the culture medium contains an aerobe, and
   the aerobe has a disrupted gene encoding a metabolic enzyme selected from the group consisting of the metabolic enzymes of glycolysis except hexokinase, thereby suppressing metabolism from a carbon source to the TCA cycle in the aerobe, and the carbon source is a nutrient selected from the group consisting of a substrate and a metabolite for glycolysis in the upstream of the metabolic enzyme disrupted, and a substrate and a metabolite for a metabolic pathway flowing into the upstream glycolysis.
[2] The culture according to [1], wherein the culture medium further contains the carbon source.
[3] The culture according to [1] or [2], wherein the aerobe allows for metabolism from a nutrient source to the TCA cycle in the presence of the nutrient source for the TCA cycle, and the nutrient source contains a nutrient selected from the group consisting of a metabolite of glycolysis in the downstream of the metabolic enzyme disrupted, a substrate and a metabolite for a metabolic pathway flowing into the downstream glycolysis, and a metabolite of the TCA cycle, and a substrate and a metabolite for a metabolic pathway flowing into the TCA cycle.
[4] The culture according to [3], wherein the culture medium further contains the nutrient source.
[5] The culture according to any one of [1] to [4], wherein the aerobe further has a disrupted gene encoding transaldolase.
[6] The culture according to any one of [1] to [5], wherein the disrupted gene encoding an enzyme of glycolysis is a gene encoding phosphofructokinase.
[7] The culture according to [6], wherein the disrupted gene encoding an enzyme of glycolysis is genes encoding phosphofructokinase A2 and A3.
[8] The culture according to [6] or [7], wherein the nutrient source is at least one molecule selected from the group consisting of glycerol, fructose 1,6-bisphosphate, glyceraldehyde 3-phosphate, dihydroxyacetone phosphate, 1,3-bisphosphoglycerate, 3-phosphoglycerate, 2-phosphoglycerate, phosphoenolpyruvate, pyruvate, acetate, NADH, malate, succinate, glutamate, α-ketoglutarate, iso-citrate, protocatechuate and amino acids except glutamate.
[9] The culture according to any one of [1] to [4], wherein the disrupted gene encoding an enzyme of glycolysis is a phosphoglycerate kinase gene.
[10] The culture according to [9], wherein the nutrient source is at least one molecule selected from the group consisting of 3-phosphoglycerate, 2-phosphoglycerate, phosphoenolpyruvate, pyruvate, acetate, NADH, malate, succinate, glutamate, α-ketoglutarate, iso-citrate, protocatechuate and amino acids except glutamate.
[11] A method for obtaining a metabolite of a metabolic pathway selected from the group consisting of glycolysis, the pentose phosphate pathway and metabolic pathways derived from these, the method comprising providing the culture according to any one of [1] to [10] and obtaining any one of the metabolites from the culture provided.
[12] A method for culturing an aerobe, wherein
   the aerobe has a disrupted gene encoding a metabolic enzyme of glycolysis selected from the group consisting of the metabolic enzymes of glycolysis except hexokinase, thereby suppressing metabolism from a carbon source to the TCA cycle in the aerobe, and the carbon source is a nutrient selected from the group consisting of a substrate and a metabolite for glycolysis in the upstream of the metabolic enzyme disrupted, and a substrate and a metabolite for a metabolic pathway flowing into the upstream glycolysis, the method comprising
   culturing the aerobe in a culture medium containing the carbon source under an aerobic condition.
[13] The method according to [12], wherein the aerobe further has a disrupted gene encoding transaldolase.
[14] The method according to [12] or [13], wherein the disrupted gene encoding an enzyme of glycolysis is a gene encoding phosphofructokinase.
[15] The method according to [14], wherein the disrupted gene encoding an enzyme of glycolysis is genes encoding phosphofructokinase A2 and A3.
[16] The method according to [14] or [15], wherein the culture medium further contains at least one molecule selected from the group consisting of glycerol, acetate, fructose 1,6-bisphosphate, glyceraldehyde 3-phosphate, dihydroxyacetone phosphate, 1,3-bisphosphoglycerate, 3-phosphoglycerate, 2-phosphoglycerate, phosphoenolpyruvate, pyruvate, NADH, malate, succinate, glutamate, α-ketoglutarate, iso-citrate, protocatechuate and amino acids except glutamate.
[17] The method according to [12] or [13], wherein the disrupted gene encoding an enzyme of glycolysis is a phosphoglycerate kinase gene.
[18] The method according to [17], wherein the culture medium further contains at least one molecule selected from the group consisting of 3-phosphoglycerate, 2-phosphoglycerate, phosphoenolpyruvate, pyruvate, acetate, NADH, malate, succinate, glutamate, α-ketoglutarate, iso-citrate, protocatechuate and amino acids except glutamate.
[19] A method for producing a metabolite of a metabolic pathway selected from the group consisting of glycolysis, the pentose phosphate pathway and metabolic pathways derived from these, in an aerobe under an aerobic condition, wherein
   the aerobe has a disrupted gene encoding a metabolic enzyme of glycolysis selected from the group consisting of the metabolic enzymes of glycolysis except hexokinase, thereby suppressing metabolism from a carbon source to the TCA cycle in the aerobe, and the carbon source is a nutrient selected from the group consisting of a substrate and a metabolite for glycolysis in the upstream of the metabolic enzyme disrupted, and a substrate and a metabolite for a metabolic pathway flowing into the upstream glycolysis, the method comprising
   culturing the aerobe in a culture medium containing the carbon source under an aerobic condition, wherein the culture medium may further contain a nutrient source, or a nutrient source and/or the carbon source may be added to the culture medium in the middle of culture,
   the carbon source is a nutrient selected from the group consisting of a substrate and a metabolite for glycolysis in the upstream of the metabolic enzyme disrupted, and a substrate and a metabolite for a metabolic pathway flowing into the upstream glycolysis, and the nutrient source contains a nutrient selected from the group consisting of a metabolite of glycolysis in the downstream of the metabolic enzyme disrupted, a substrate and a metabolite for a metabolic pathway flowing into the downstream glycolysis, a metabolite of the TCA cycle, and a substrate and a metabolite for a metabolic pathway flowing into the TCA cycle.
[20] The method according to [19], wherein the aerobe further has a disrupted gene encoding transaldolase.
[21] The method according to [19] or [20], wherein the disrupted gene encoding an enzyme of glycolysis is a gene encoding phosphofructokinase.
[22] The method according to [21], wherein the disrupted gene encoding an enzyme of glycolysis is genes encoding phosphofructokinase A2 and A3.
[23] The method according to [21] or [22], wherein the culture medium further contains at least one molecule selected from the group consisting of glycerol, acetate, fructose 1,6-bisphosphate, glyceraldehyde 3-phosphate, dihydroxyacetone phosphate, 1,3-bisphosphoglycerate, 3-phosphoglycerate, 2-phosphoglycerate, phosphoenolpyruvate, pyruvate, NADH, malate, succinate, glutamate, α-ketoglutarate, iso-citrate, protocatechuate and amino acids except glutamate.
[24] The method according to [19] or [20], wherein the disrupted gene encoding an enzyme of glycolysis is a phosphoglycerate kinase gene.
[25] The method according to [24], wherein the culture medium further contains at least one molecule selected from the group consisting of 3-phosphoglycerate, 2-phosphoglycerate, phosphoenolpyruvate, pyruvate, acetate, NADH, malate, succinate, glutamate, α-ketoglutarate, iso-citrate, protocatechuate and amino acids except glutamate.
[26] A method for proliferating an aerobe under an aerobic condition, wherein
   the aerobe has a disrupted gene encoding a metabolic enzyme of glycolysis selected from the group consisting of the metabolic enzymes of glycolysis except hexokinase, thereby suppressing metabolism from a carbon source to the TCA cycle in the aerobe, and the carbon source is a nutrient selected from the group consisting of a substrate and a metabolite for glycolysis in the upstream of the metabolic enzyme disrupted, and a substrate and a metabolite for a metabolic pathway flowing into the upstream glycolysis, the method comprising
   culturing the aerobe in the presence of a nutrient source under an aerobic condition, wherein the culture medium may further contain the carbon source or a nutrient source and/or the carbon source may be added to the culture medium in the middle of culture, and
   the nutrient source contains a nutrient selected from the group consisting of a metabolite of glycolysis in the downstream of the metabolic enzyme disrupted, a substrate and a metabolite for a metabolic pathway flowing into the downstream glycolysis, a metabolite of the TCA cycle, and a substrate and a metabolite for a metabolic pathway flowing into the TCA cycle.
[27] An actinomycete having disrupted genes encoding phosphofructokinase A2 and A3.
[28] The actinomycete according to [27], wherein the actinomycete further has a disrupted gene encoding transaldolase.
[29] An actinomycete having a disrupted gene encoding phosphoglycerate kinase.

[1A] A culture comprising a culture medium used in culturing under an aerobic condition, wherein
   the culture medium contains an aerobe, and
   the aerobe has a disrupted gene encoding a metabolic enzyme of glycolysis selected from the group consisting of the metabolic enzymes of glycolysis except hexokinase, thereby suppressing metabolism from glucose to the TCA cycle in the aerobe.
[2A] The culture according to [1A], wherein the culture medium further contains a carbon source, and the carbon source is a nutrient selected from the group consisting of a substrate and a metabolite for glycolysis in the upstream of the metabolic enzyme disrupted, and a substrate and a metabolite for a metabolic pathway flowing into the upstream glycolysis.
[3A] The culture according to [1A] or [2A], wherein the aerobe allows for metabolism from a nutrient source to the TCA cycle in the presence of the nutrient source for the TCA cycle, and the nutrient source contains a nutrient selected from the group consisting of a metabolite of glycolysis in the downstream of the metabolic enzyme disrupted, a substrate and a metabolite for a metabolic pathway flowing into the downstream glycolysis, and a metabolite of the TCA cycle, and a substrate and a metabolite for a metabolic pathway flowing into the TCA cycle.
[4A] The culture according to [3A], wherein the culture medium further contains the nutrient source.
[5A] The culture according to any one of [1A] to [4A], wherein the aerobe further has a disrupted gene encoding transaldolase.
[6A] The culture according to any one of [1A] to [5A], wherein the disrupted gene encoding an enzyme of glycolysis is a gene encoding phosphofructokinase.
[7A] The culture according to [6A], wherein the disrupted gene encoding an enzyme of glycolysis is genes encoding phosphofructokinase A2 and A3.
[8A] The culture according to [6A] or [7A], wherein the nutrient source is at least one molecule selected from the group consisting of glycerol, fructose 1,6-bisphosphate, glyceraldehyde 3-phosphate, dihydroxyacetone phosphate, 1,3-bisphosphoglycerate, 3-phosphoglycerate, 2-phosphoglycerate, phosphoenolpyruvate, pyruvate, acetate, NADH, malate, succinate, glutamate, α-ketoglutarate, iso-citrate, protocatechuate and amino acids except glutamate.
[9A] The culture according to any one of [1A] to [4A], wherein the disrupted gene encoding an enzyme of glycolysis is a phosphoglycerate kinase gene.
[10A] The culture according to [9A], wherein the nutrient source is at least one molecule selected from the group consisting of 3-phosphoglycerate, 2-phosphoglycerate, phosphoenolpyruvate, pyruvate, acetate, NADH, malate, succinate, glutamate, α-ketoglutarate, iso-citrate, protocatechuate and amino acids except glutamate.
[11A] A method for obtaining a metabolite of a metabolic pathway selected from the group consisting of glycolysis, the pentose phosphate pathway and metabolic pathways derived from these, the method comprising providing the culture according to [1A] to [10A] and obtaining any one of the metabolites from the culture provided.
[12A] A method for culturing an aerobe, wherein
   the aerobe has a disrupted gene encoding a metabolic enzyme of glycolysis selected from the group consisting of the metabolic enzymes of glycolysis except hexokinase, thereby suppressing metabolism from glucose to the TCA cycle in the aerobe, the method comprising
   culturing the aerobe in a culture medium containing a carbon source under an aerobic condition, wherein the carbon source is a nutrient selected from the group consisting of a substrate and a metabolite for glycolysis in the upstream of the metabolic enzyme disrupted, and a substrate and a metabolite for a metabolic pathway flowing into the upstream glycolysis.
[13A] The method according to [12A], wherein the aerobe further has a disrupted gene encoding transaldolase.
[14A] The method according to [12A] or [13A], wherein the disrupted gene encoding an enzyme of glycolysis is a gene encoding phosphofructokinase.
[15A] The method according to [14A], wherein the disrupted gene encoding an enzyme of glycolysis is genes encoding phosphofructokinase A2 and A3.
[16A] The method according to [14A] or [15A], wherein the culture medium further contains at least one molecule selected from the group consisting of glycerol, acetate, fructose 1,6-bisphosphate, glyceraldehyde 3-phosphate, dihydroxyacetone phosphate, 1,3-bisphosphoglycerate, 3-phosphoglycerate, 2-phosphoglycerate, phosphoenolpyruvate, pyruvate, NADH, malate, succinate, glutamate, α-ketoglutarate, iso-citrate, protocatechuate and amino acids except glutamate.
[17A] The method according to [12A] or [13A], wherein the disrupted gene encoding an enzyme of glycolysis is a phosphoglycerate kinase gene.
[18A] The method according to [17A], wherein the culture medium further contains at least one molecule selected from the group consisting of 3-phosphoglycerate, 2-phosphoglycerate, phosphoenolpyruvate, pyruvate, acetate, NADH, malate, succinate, glutamate, α-ketoglutarate, iso-citrate, protocatechuate and amino acids except glutamate.
[19A] A method for producing a metabolite of a metabolic pathway selected from the group consisting of glycolysis, the pentose phosphate pathway and metabolic pathways derived from these, in an aerobe under an aerobic condition, wherein
   the aerobe has a disrupted gene encoding a metabolic enzyme of glycolysis selected from the group consisting of the metabolic enzymes of glycolysis except hexokinase, thereby suppressing metabolism from glucose to the TCA cycle in the aerobe, the method comprising
   culturing the aerobe in a culture medium containing a carbon source under an aerobic condition, wherein the culture medium may further contain a nutrient source, or a nutrient source and/or a carbon source may be added to the culture medium in the middle of culture, and
   the carbon source is a nutrient selected from the group consisting of a substrate and a metabolite for glycolysis in the upstream of the metabolic enzyme disrupted, and a substrate and a metabolite for a metabolic pathway flowing into the upstream glycolysis, and the nutrient source contains a nutrient selected from the group consisting of a metabolite of glycolysis in the downstream of the metabolic enzyme disrupted, a substrate and a metabolite for a metabolic pathway flowing into the downstream glycolysis, a metabolite of the TCA cycle, and a substrate and a metabolite for a metabolic pathway flowing into the TCA cycle.
[20A] The method according to [19A], wherein the aerobe further has a disrupted gene encoding transaldolase.
[21A] The method according to [19A] or [20A], wherein the disrupted gene encoding an enzyme of glycolysis is a gene encoding phosphofructokinase.
[22A] The method according to [21A], wherein the disrupted gene encoding an enzyme of glycolysis is genes encoding phosphofructokinase A2 and A3.
[23A] The method according to [21A] or [22A], wherein the culture medium further contains at least one molecule selected from the group consisting of glycerol, acetate, fructose 1,6-bisphosphate, glyceraldehyde 3-phosphate, dihydroxyacetone phosphate, 1,3-bisphosphoglycerate, 3-phosphoglycerate, 2-phosphoglycerate, phosphoenolpyruvate, pyruvate, NADH, malate, succinate, glutamate, α-ketoglutarate, iso-citrate, protocatechuate and amino acids except glutamate.
[24A] The method according to [19A] or [20A], wherein the disrupted gene encoding an enzyme of glycolysis is a phosphoglycerate kinase gene.
[25A] The method according to [24A], wherein the culture medium further contains at least one molecule selected from the group consisting of 3-phosphoglycerate, 2-phosphoglycerate, phosphoenolpyruvate, pyruvate, acetate, NADH, malate, succinate, glutamate, α-ketoglutarate, iso-citrate, protocatechuate and amino acids except glutamate.
[26A] A method for proliferating an aerobe under an aerobic condition, wherein
   the aerobe has a disrupted gene encoding a metabolic enzyme of glycolysis selected from the group consisting of the metabolic enzymes of glycolysis except hexokinase, thereby suppressing metabolism from glucose to the TCA cycle in the aerobe, the method comprising
   culturing the aerobe in the presence of a nutrient source under an aerobic condition, wherein the culture medium may further contain a carbon source, or a nutrient source and/or a carbon source may be added to the culture medium in the middle of culture, and
   the carbon source is a nutrient selected from the group consisting of a substrate and a metabolite for glycolysis in the upstream of the metabolic enzyme disrupted, and a substrate and a metabolite for a metabolic pathway flowing into the upstream glycolysis, and the nutrient source contains a nutrient selected from the group consisting of a metabolite of glycolysis in the downstream of the metabolic enzyme disrupted, a substrate and a metabolite for a metabolic pathway flowing into the downstream glycolysis, a metabolite of the TCA cycle, and a substrate and a metabolite for a metabolic pathway flowing into the TCA cycle.

According to the present invention, the following inventions are further provided.
[1B] A culture comprising a culture medium used in culturing under an aerobic condition, wherein
   the culture medium contains an aerobe and a nutrient source, wherein the nutrient source contains a nutrient source in an amount sufficient for the aerobe to proliferate under an aerobic environment,
   the aerobe has a disrupted gene encoding a metabolic enzyme selected from the group consisting of the metabolic enzymes of glycolysis except hexokinase, thereby suppressing metabolism from a carbon source to the TCA cycle in the aerobe, and the carbon source is a nutrient selected from the group consisting of a substrate and a metabolite for glycolysis in the upstream of the metabolic enzyme disrupted, and a substrate and a metabolite for a metabolic pathway flowing into the upstream glycolysis, and
   the aerobe allows for metabolism from the nutrient source to the TCA cycle in the presence of the nutrient source for the TCA cycle, and the nutrient source contains a nutrient selected from the group consisting of a metabolite of glycolysis in the downstream of the metabolic enzyme disrupted, a substrate and a metabolite for a metabolic pathway flowing into the downstream glycolysis, a metabolite of the TCA cycle, and a substrate and a metabolite for a metabolic pathway flowing into the TCA cycle.
[2B] The culture according to [1B], wherein the culture medium further contains the carbon source.
[3B] The culture according to [1B] or [2B], wherein at least one of the nutrient sources has a concentration of 2.0 mM or more.
[4B] The culture according to [3B], wherein at least one of the nutrient sources has a concentration of 3.0 mM or more.
[5B] The culture according to any one of [1B] to [4B], wherein the aerobe further has a disrupted gene encoding transaldolase.
[6B] The culture according to any one of [1B] to [5B], wherein the disrupted gene encoding an enzyme of glycolysis is a gene encoding phosphofructokinase.
[7B] The culture according to [6B], wherein the disrupted gene encoding an enzyme of glycolysis is genes encoding phosphofructokinase A2 and A3.
[8B] The culture according to [6B] or [7B], wherein the nutrient source is at least one molecule selected from the group consisting of glycerol, fructose 1,6-bisphosphate, glyceraldehyde 3-phosphate, dihydroxyacetone phosphate, 1,3-bisphosphoglycerate, 3-phosphoglycerate, 2-phosphoglycerate, phosphoenolpyruvate, pyruvate, acetate, NADH, malate, succinate, glutamate, α-ketoglutarate, iso-citrate, protocatechuate and amino acids except glutamate.
[9B] The culture according to any one of [1B] to [4B], wherein the disrupted gene encoding an enzyme of glycolysis is a phosphoglycerate kinase gene.
[10B] The culture according to [9B], wherein the nutrient source is at least one molecule selected from the group consisting of 3-phosphoglycerate, 2-phosphoglycerate, phosphoenolpyruvate, pyruvate, acetate, NADH, malate, succinate, glutamate, α-ketoglutarate, iso-citrate, protocatechuate and amino acids except glutamate.
[11B] A method for obtaining a metabolite of a metabolic pathway selected from the group consisting of glycolysis, the pentose phosphate pathway and metabolic pathways derived from these, the method comprising providing the culture according to any one of [1B] to [10B] and obtaining any one of the metabolites from the culture provided.
[12B] A method for culturing an aerobe, wherein
   the aerobe has a disrupted gene encoding a metabolic enzyme of glycolysis selected from the group consisting of the metabolic enzymes of glycolysis except hexokinase, thereby suppressing metabolism from a carbon source to the TCA cycle in the aerobe, and
   the carbon source is a nutrient selected from the group consisting of a substrate and a metabolite for glycolysis in the upstream of the metabolic enzyme disrupted, and a substrate and a metabolite for a metabolic pathway flowing into the upstream glycolysis, the method comprising
   culturing the aerobe in a culture medium containing the carbon source and a nutrient source under an aerobic condition, wherein the nutrient source contains a nutrient source in an amount sufficient for the aerobe to proliferate under an aerobic environment.
[13B] The method according to [12B], wherein the aerobe further has a disrupted gene encoding transaldolase.
[14B] The method according to [12B] or [13B], wherein the disrupted gene encoding an enzyme of glycolysis is a gene encoding phosphofructokinase.
[15B] The method according to [14B], wherein the disrupted gene encoding an enzyme of glycolysis is genes encoding phosphofructokinase A2 and A3.
[16B] The method according to [14B] or [15B], wherein the culture medium further contains at least one molecule selected from the group consisting of glycerol, acetate, fructose 1,6-bisphosphate, glyceraldehyde 3-phosphate, dihydroxyacetone phosphate, 1,3-bisphosphoglycerate, 3-phosphoglycerate, 2-phosphoglycerate, phosphoenolpyruvate, pyruvate, NADH, malate, succinate, glutamate, α-ketoglutarate, iso-citrate, protocatechuate and amino acids except glutamate.
[17B] The method according to [12B] or [13B], wherein the disrupted gene encoding an enzyme of glycolysis is a phosphoglycerate kinase gene.
[18B] The method according to [17B], wherein the culture medium further contains at least one molecule selected from the group consisting of 3-phosphoglycerate, 2-phosphoglycerate, phosphoenolpyruvate, pyruvate, acetate, NADH, malate, succinate, glutamate, α-ketoglutarate, iso-citrate, protocatechuate and amino acids except glutamate.
[19B] A method for producing a metabolite of a metabolic pathway selected from the group consisting of glycolysis, the pentose phosphate pathway and metabolic pathways derived from these, in an aerobe under an aerobic condition, wherein
   the aerobe has a disrupted gene encoding a metabolic enzyme of glycolysis selected from the group consisting of the metabolic enzymes of glycolysis except hexokinase, thereby suppressing metabolism from a carbon source to the TCA cycle in the aerobe, and the carbon source is a nutrient selected from the group consisting of a substrate and a metabolite for glycolysis in the upstream of the metabolic enzyme disrupted, and a substrate and a metabolite for a metabolic pathway flowing into the upstream glycolysis, the method comprising
   culturing the aerobe in a culture medium containing the carbon source under an aerobic condition, wherein the culture medium may further contain a nutrient source, or a nutrient source and/or the carbon source may be added to the culture medium in the middle of culture, and the nutrient source contains a nutrient source in an amount sufficient for the aerobe to proliferate under an aerobic environment, and
   the nutrient source contains a nutrient selected from the group consisting of a metabolite of glycolysis in the downstream of the metabolic enzyme disrupted, a substrate and a metabolite for a metabolic pathway flowing into the downstream glycolysis, a metabolite of the TCA cycle, and a substrate and a metabolite for a metabolic pathway flowing into the TCA cycle.
[20B] The method according to [19B], wherein the aerobe further has a disrupted gene encoding transaldolase.
[21B] The method according to [19B] or [20B], wherein the disrupted gene encoding an enzyme of glycolysis is a gene encoding phosphofructokinase.
[22B] The method according to [21B], wherein the disrupted gene encoding an enzyme of glycolysis is genes encoding phosphofructokinase A2 and A3.
[23B] The method according to [21B] or [22B], wherein the culture medium further contains at least one molecule selected from the group consisting of glycerol, acetate, fructose 1,6-bisphosphate, glyceraldehyde 3-phosphate, dihydroxyacetone phosphate, 1,3-bisphosphoglycerate, 3-phosphoglycerate, 2-phosphoglycerate, phosphoenolpyruvate, pyruvate, NADH, malate, succinate, glutamate, α-ketoglutarate, iso-citrate, protocatechuate and amino acids except glutamate.
[24B] The method according to [19B] or [20B], wherein the disrupted gene encoding an enzyme of glycolysis is a phosphoglycerate kinase gene.
[25B] The method according to [24B], wherein the culture medium further contains at least one molecule selected from the group consisting of 3-phosphoglycerate, 2-phosphoglycerate, phosphoenolpyruvate, pyruvate, acetate, NADH, malate, succinate, glutamate, α-ketoglutarate, iso-citrate, protocatechuate and amino acids except glutamate.
[26B] A method for proliferating an aerobe under an aerobic condition, wherein
   the aerobe has a disrupted gene encoding a metabolic enzyme of glycolysis selected from the group consisting of the metabolic enzymes of glycolysis except hexokinase, thereby suppressing metabolism from a carbon source to the TCA cycle in the aerobe, and
   the carbon source is a nutrient selected from the group consisting of a substrate and a metabolite for glycolysis in the upstream of the metabolic enzyme disrupted, and a substrate and a metabolite for a metabolic pathway flowing into the upstream glycolysis, the method comprising
   culturing the aerobe in the presence of a nutrient source under an aerobic condition, wherein the nutrient source contains a nutrient source in an amount sufficient for the aerobe to proliferate under an aerobic environment, and
   the nutrient source contains a nutrient selected from the group consisting of a metabolite of glycolysis in the downstream of the metabolic enzyme disrupted, a substrate and a metabolite for a metabolic pathway flowing into the downstream glycolysis, a metabolite of the TCA cycle, and a substrate and a metabolite for a metabolic pathway flowing into the TCA cycle.
[27B] An actinomycete having disrupted genes encoding phosphofructokinase A2 and A3.
[28B] The actinomycete according to [27B], wherein the actinomycete further has a disrupted gene encoding transaldolase.
[29B] An actinomycete having a disrupted gene encoding phosphoglycerate kinase.

According to the method of the present invention, the amount of a substance to be produced can be increased by enhancing availability of a carbon source to substance production. The amount of a substance to be produced can be further increased by reducing the decrease of cell proliferation that occurs at this time. According to the present invention, the method of the invention is advantageous in proliferating cells when a substance is produced by culturing. Examples of the culture includes pre-preculture, preculture and main culture, which are carried out for the substance production.

### Brief Description of Drawings

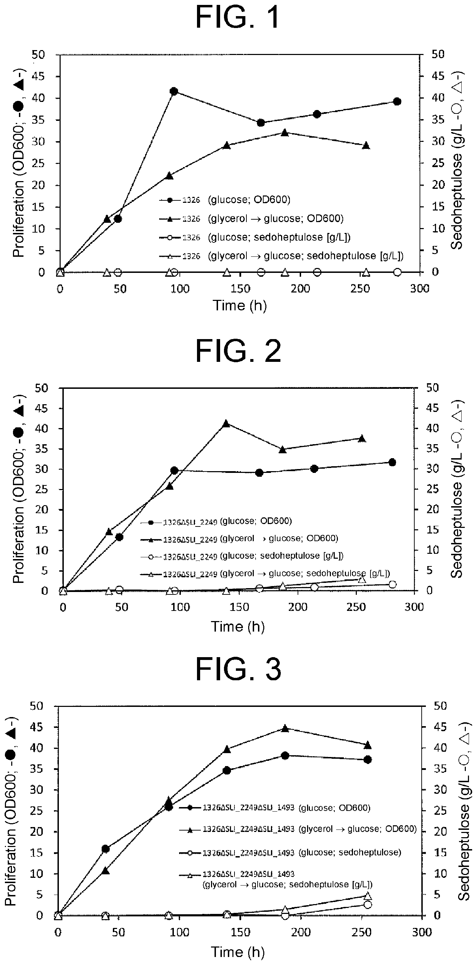

[Figure 1] Figure 1 shows the results of a culture experiment of *Streptomyces lividans* 1326 strain. The horizontal axis represents culture time (h); whereas, the vertical axis represents the amount of cells calculated from OD600. A solid circle shows the result of the case where 1326 strain was cultured in the presence of glucose; an open circle shows the amount of sedoheptulose obtained in the case; a solid triangle shows the case where culture was initiated in TSB medium containing glycerol and glucose was added before glycerol was used up; and an open triangle shows the amount of sedoheptulose obtained in the case.
[Figure 2] Figure 2 shows the results of *Streptomyces lividans* 1326ΔSLI_2249.
[Figure 3] Figure 3 shows the results of *Streptomyces lividans* 1326ΔSLI_2249ΔSLI1493.
[Figure 4] Figure 4 shows the results of *Streptomyces lividans* 1326ΔSLI_2249ΔSLI_1493ΔSLI_5695.
[Figure 5] Figure 5 shows the amount of trehalose produced per unit cell when *Streptomyces lividans* 1326 strain and *Streptomyces lividans* 1326ΔSLI_2260 strain were used. An open circle shows the result of the case where 1326 strain was cultured in TSB medium; an open triangle shows the result of the case where 1326 strain was cultured in 5X TSB medium; a solid circle shows the result of the case where 1326ΔSLI_2260 strain was cultured in TSB medium; and a solid triangle shows the result of the case where 1326ΔSLI_2260 strain was cultured in 5X TSB medium.
[Figure 6] Figure 6 shows the effect of protocatechuate on proliferation of *Streptomyces lividans* 1326ΔSLI_2260 strain cultured in the presence of glucose.
[Figure 7] Figure 7 shows the effects of amino acids (alanine, serine and glutamate) on proliferation of *Streptomyces lividans* 1326ΔSLI_2260 strain.
[Figure 8] Figure 8 shows the effect of an amino acid (alanine) on proliferation of *Streptomyces lividans* 1326ΔSLI_2260 strain cultured in the presence of glycerol.
[Figure 9] Figure 9 shows the effect of succinate on proliferation of *Streptomyces lividans* 1326ΔSLI_2260 strain cultured in the presence of glycerol.
[Figure 10] Figure 10 shows proliferation of *Streptomyces lividans* 1326ΔSLI_2260 strain in the presence of xylose and an amino acid (alanine).
[Figure 11] Figure 11 shows the production amount of xylulose per unit cell of *Streptomyces lividans* 1326ΔSLI_2260 strain cultured in the presence of xylose and an amino acid (alanine).
[Figure 12] Figure 12 shows the production amount of xylitol produced per unit cell of *Streptomyces lividans* 1326ΔSLI_2260 strain cultured in the presence of xylose and an amino acid (alanine).

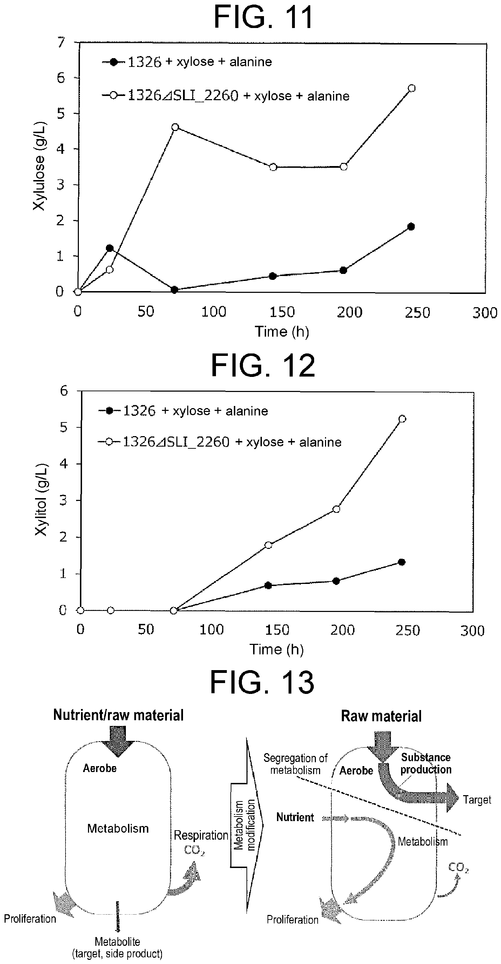

[Figure 13] Figure 13 is a conceptual view illustrating a manner how to modify metabolism in accordance with the present invention. In the usual case (left), nutrients including a carbon source and a nutrient source, are used for not only cell proliferation but also substance production. In contrast, in the case of the present invention (right), a metabolic enzyme of glycolysis is disrupted, thereby preventing a raw material (carbon source) for substance production from being used in proliferation of cells; at the same time, a nutrient (nutrient source) required for proliferation is separately supplied to cells, thereby promoting cell proliferation and/or promoting substance production from the raw material. In this way, substance production and cell proliferation do not compete for a nutrient.
[Figure 14] Figure 14 shows glycolysis, the pentose phosphate pathway and examples of metabolic pathways derived from these; and examples of metabolites of these pathways.
[Figure 15] Figure 15 shows examples of nutrients serving as a nutrient source and a carbon source in glycolysis and the pentose phosphate pathway.

### Detailed Description of the Invention

In the specification, the "aerobe" refers to a microorganism having a mechanism of consuming oxygen to produce energy. The aerobe utilizes oxygen in order to obtain energy from a sugar, a lipid, and the like. Examples of the aerobe include facultative aerobe and obligate aerobe. The facultative aerobe can utilize oxygen but anaerobically produce energy. Examples of the aerobe herein can include microorganisms classified as anaerobe such as facultative anaerobe and the like as long as they satisfy the above definition. Examples of the facultative aerobe include yeasts, bacteria and molds. An aerobe in which a metabolic enzyme is disrupted is established as a strain. Accordingly, the aerobe having a metabolic enzyme disrupted will be sometimes simply referred to as a metabolic enzyme-disrupted strain.

In the specification, "actinomycete" refers to a bacterium belonging to the phylum *Actinobacteria* (actinomycetes). Examples of actinomycetes include, but are not particularly limited to, bacteria of the genera *Streptomyces, Actinomyces, Mycobacterium* and *Corynebacterium.* Actinomycete is a facultative aerobe but capable of producing energy in an anaerobic condition. However, for producing a substance, actinomycete is cultured under an aerobic condition. Examples of actinomycete of the genus *Streptomyces* include *Streptomyces lividans, Streptomyces violaceoruber, Streptomyces coelicolor, Streptomyces avermitilis, Streptomyces griseus, Streptomyces albus* and *Streptomyces albulus.*

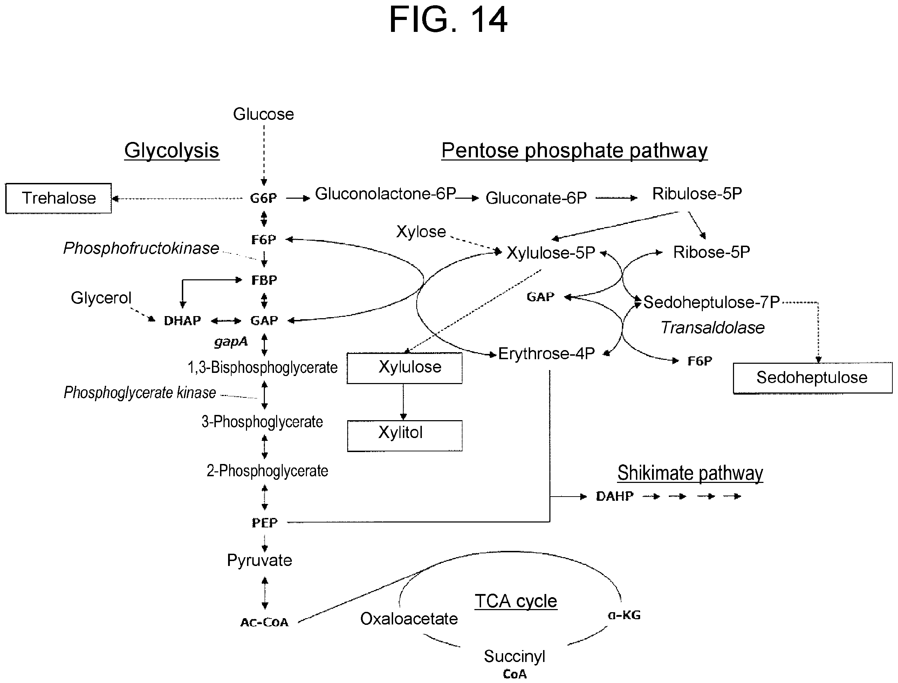

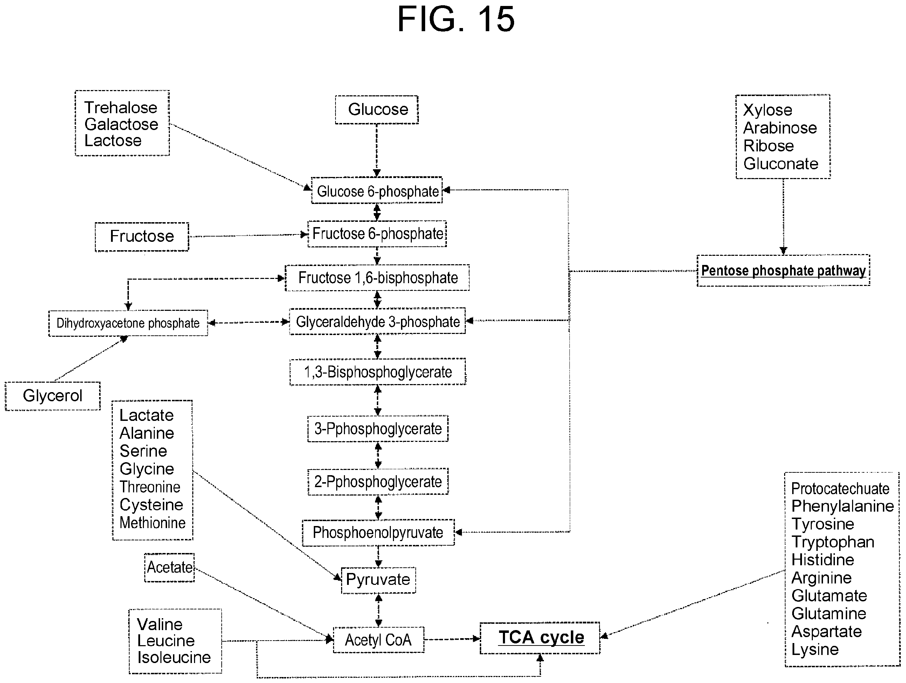

In the specification, the "aerobic condition" refers to a condition where an aerobe aerobically respirates. Aerobic respiration is mediated by the metabolic system including glycolysis, citrate cycle and electron transport system. Due to the metabolic system, usually, a sugar consumes oxygen to produce carbon dioxide, water and ATP serving as energy. Glycolysis is a pathway for producing pyruvate from glucose via glucose 6-phosphate as shown in Figures 14 and 15. The pyruvate produced in glycolysis is converted into acetyl CoA by a pyruvate dehydrogenase complex and introduced to the citrate cycle (for convenience, the passage up to production of acetyl CoA is classified in glycolysis in this specification). Acetyl CoA is thereafter metabolized in the citrate cycle. The citrate cycle is also called the TCA cycle. In the citrate cycle, for example, NADH, FADH₂ and GTP are produced. In the electron transport system, NADH and FADH₂ are oxidized to produce ATP. When an aerobe is cultured under an aerobic condition, ATP is produced from a sugar in the metabolic system including glycolysis, citrate cycle and electron transport system. In contrast, the "anaerobic condition" refers to a condition in which an anaerobe anaerobically produces energy and proliferates. Those skilled in the art can appropriately culture an aerobe under an aerobic condition.

In the present invention, metabolization of glucose in glycolysis and the TCA cycle is inhibited. Inhibition of glucose metabolization can be made by disrupting at least one of the glycolytic enzymes for metabolizing glucose to acetyl CoA. Examples of the glycolytic enzyme (including metabolic enzymes) include metabolic enzymes responsible for the following 10 steps shown from the top to the bottom in Figure 14.
- Hexokinase (EC 2.7.1.1) is a metabolic enzyme responsible for metabolization of glucose into glucose 6-phosphate (G6P) (step 1), (note that glucose 6-phosphate can be used as a carbon source for the pentose phosphate pathway),
- Glucose 6-phosphate isomerase (EC 5.3.1.9) is a metabolic enzyme responsible for metabolization of glucose 6-phosphate into fructose 6-phosphate (F6P) (step 2),
- Phosphofructokinase (EC 2.7.1.11) is a metabolic enzyme responsible for metabolization of fructose 6-phosphate into fructose 1,6-bisphosphate (FBP) (step 3), (note that, since fructose 1,6-bisphosphate is only metabolized in glycolysis, glycolysis alone can be manipulated by disruption of phosphofructokinase),
- Fructose 1,6-bisphosphate aldolase (EC 4.1.2.13) is a metabolic enzyme responsible for metabolization of fructose 1,6-bisphosphate into glyceraldehyde 3-phosphate (GAP) and dihydroxyacetone phosphate (DHAP) (step 4),
- Triosephosphate isomerase (EC 5.3.1.1) is a metabolic enzyme responsible for conversion of dihydroxyacetone phosphate into glyceraldehyde 3-phosphate (step 5),
- Glyceraldehyde 3-phosphonate dehydrogenase (EC 1.2.1.12) (gapA) is a metabolic enzyme responsible for metabolization of glyceraldehyde 3-phosphate into 1,3-bisphosphoglycerate (step 6),
- Phosphoglycerate kinase (EC 2.7.2.3) is a metabolic enzyme responsible for metabolization of 1,3-bisphosphoglycerate into 3-phosphoglycerate (step 7),
- Phosphoglycerate mutase (EC 5.4.2.1) is a metabolic enzyme responsible for metabolization of 3-phosphoglycerate into 2-phosphoglycerate (step 8),
- Phosphopyruvate hydratase (EC 4.2.1.11) is a metabolic enzyme responsible for metabolization of 2-phosphoglycerate into phosphoenolpyruvate (PEP) (step 9), and
- Pyruvate kinase (EC 2.7.1.40) is a metabolic enzyme responsible for metabolization of phosphoenolpyruvate into pyruvate (step 10). Note that pyruvate is thereafter converted into acetyl CoA by pyruvate dehydrogenase (EC 1.2.1.51) in glycolysis. Phosphoenolpyruvate reacts with a carbonate ion into oxaloacetate and inorganic phosphate by phosphoenolpyruvate carboxylase (EC 4.1.1.31). Pyruvate reacts with a carbonate ion into oxaloacetate by pyruvate carboxylase (EC 6.4.1.1). Acetyl CoA (Ac-CoA) and oxaloacetate are introduced to the TCA cycle. The enzymatic reactions of glycolysis are sequentially described from the upstream to the downstream in the above. Although the types of metabolic enzymes may vary depending on the species, basically the same metabolic pathway is present in individual organisms. An aerobe has glycolysis, and metabolic enzymes responsible for the steps corresponding to steps 1 to 10 are present. Accordingly, if any one of the steps 1 to 10 of glycolysis in an aerobe is desired to stop, the metabolic enzyme responsible for metabolism of the desired step may be disrupted.

In the present invention, consumption of glucose or glucose 6-phosphate in the TCA cycle can be inhibited by disrupting at least one of the metabolic enzymes (except hexokinase) in the metabolic pathway of glycolysis from glucose to pyruvate. In this manner, consumption of glucose and glucose 6-phosphate by cells can be prevented. If so, in the cells, the amounts of glucose and glucose 6-phosphate, which are to be consumed in the pentose phosphate pathway for producing substances, can be increased. Whereas, in the present invention, a metabolite and the like (metabolite, substance to be converted *in vivo* into the metabolite, an amino acid, and the like) in the downstream of the metabolic enzyme disrupted in the above is supplied, which allows the TCA cycle to produce energy. Thereby, proliferation of cells, which reduced by disruption of a metabolic enzyme, can be retrieved.

Accordingly, in the present invention, (i) consumption of glucose or glucose 6-phosphate in the TCA cycle is inhibited by disrupting at least one of the metabolic enzymes (except hexokinase) of the metabolic pathway of glycolysis from glucose to acetyl CoA, and (ii) energy can be supplied to cells by providing an energy source for the TCA cycle.

In the specification, the "carbon source" refers to a nutrient, which is to be consumed in glycolysis and the downstream thereof in wild aerobe under an aerobic condition but consumption of which is decreased or stopped in glycolysis and the downstream thereof in the metabolic enzyme-disrupted strain. In the metabolic enzyme-disrupted strain, the amount of the nutrient to be used for producing energy is reduced and the nutrient is likely to be used for producing a substance. The "carbon source" used herein can be referred to a nutrient whose metabolism into acetyl CoA is inhibited particularly in glycolysis and which is to be consumed in glycolysis in the upstream of the disrupted metabolic enzyme or in the pentose phosphate pathway. A carbon source includes a metabolite of a metabolic pathway flowing into glycolysis. This is because the metabolite of a metabolic pathway flowing into glycolysis (particularly, a metabolite of a metabolic pathway flowing into upstream of the metabolic enzyme disrupted) flows into glycolysis and can be supplied as a carbon source. If a plurality of metabolic enzymes are disrupted, a carbon source may be a nutrient present in the upstream of the metabolic enzyme disrupted present in the most downstream or a nutrient present in the upstream of the metabolic enzyme disrupted present in the most upstream. In an embodiment, as a carbon source, although it is not particularly limited, for example, glucose can be preferably used.

In the specification, the "nutrient source" refers to a nutrient that can induce metabolism in the TCA cycle in a metabolic enzyme-disrupted strain. In the metabolic enzyme-disrupted strain, utilization of a carbon source for energy production (for example, energy production by production of pyruvate and supply of a metabolite to the TCA cycle) is low or ceased. Accordingly, supplying a carbon source to the metabolic enzyme-disrupted strain is not sufficiently effective or utterly ineffective for the metabolic enzyme-disrupted strain to proliferate and produce energy. In contrast, if a metabolite that can be used for energy production is supplied to the metabolic enzyme-disrupted strain, the strain can produce energy and further proliferate. Particularly, in the specification, the "nutrient source" refers to a nutrient for driving energy production in the electron transport system of an aerobe having a disrupted metabolic enzyme. Examples of the nutrient source include, but are not particularly limited to, any one of metabolites of glycolysis in the downstream of a disrupted metabolic enzyme, an amino acid, NADH, FADH₂, GTP, metabolites of the TCA cycle (such as NADH, malate, succinate, glutamate, α-ketoglutarate, iso-citrate and protocatechuate). These can be preferably used. Note that amino acids such as alanine, serine, glycine, threonine, cysteine and methionine are used, via pyruvate, as nutrient sources for the TCA cycle; that protocatechuate and aromatic amino acids are used, via succinyl CoA, as nutrient sources for the TCA cycle; that acetate and amino acids such as valine, leucine and isoleucine are used, via acetyl CoA (Ac-CoA), as nutrient sources for the TCA cycle; that amino acids such as histidine, arginine, glutamine and glutamate are used, via α-ketoglutarate (α-KG), as nutrient sources for the TCA cycle; and that amino acids such as aspartate and lysine can be used, via oxaloacetate, as nutrient sources for the TCA cycle. Nutrient sources includes a metabolite of a metabolic pathway (particularly, a metabolic pathway flowing into downstream of a disrupted metabolic pathway) flowing into glycolysis. This is because the metabolite of a metabolic pathway (particularly, a metabolic pathway flowing into downstream of a disrupted metabolic pathway) flowing into glycolysis can supply a nutrient source. When a plurality of metabolic enzymes are disrupted, a nutrient source may be, for example, a nutrient in the downstream of the metabolic enzyme disrupted in the most downstream or a nutrient in the downstream of the metabolic enzyme disrupted present in the most upstream.

As described above, in the specification, a nutrient is described distinctively as a "carbon source " and a "nutrient source". Whether a nutrient serves as a carbon source or a nutrient source (relatively) varies depending on the disrupted metabolic enzyme. This can be understood by those skilled in the art.

In the specification, the "metabolic pathway derived" refers to a metabolic pathway in the downstream of a metabolic pathway or a metabolic pathway branched from a metabolic pathway. For example, a metabolic pathway derived from the pentose phosphate pathway is a metabolic pathway in the downstream of the pentose phosphate pathway or a metabolic pathway branched from a metabolic pathway of the pentose phosphate pathway. If the amount of a carbon source consumed by the pentose phosphate pathway increases, a yield of a metabolite of the pentose phosphate pathway increases. As a result, it is expected that the yield of a metabolite of the metabolic pathway (for example, shikimate pathway) derived from the pentose phosphate pathway increases too. For example, a metabolic pathway derived from glycolysis is a metabolic pathway present in the downstream of glycolysis and branched from an upstream portion of a disrupted metabolic enzyme. In glycolysis, if the consumption amount of a carbon source in the upstream of a disrupted metabolic enzyme increases, the yield of a metabolite in the upstream of glycolysis increases. As a result, it is expected that the yield of a metabolite in a metabolic pathway derived from glycolysis increases too.

In the specification, the "amino acid" refers to an amino acid of an aerobe, more specifically, an amino acid which can be used by an aerobe as a nutrient source. Examples of the amino acid include, but are not limited to, histidine, isoleucine, leucine, lysin, methionine, phenylalanine, threonine, tryptophan, valine, arginine, cysteine, glutamine, glycine, proline, tyrosine, alanine, aspartate, asparagine, glutamate and serine.

The aerobe used in the present invention has a disrupted gene encoding a metabolic enzyme of glycolysis selected from the group consisting of the metabolic enzymes of glycolysis except hexokinase. Due to disruption, the amount of glucose to be used in cellular energy production reduces or glucose cannot be used for cellular energy production.

Accordingly, (A) according to the present invention, there is provided a method for proliferating an aerobe under an aerobic condition, wherein
the aerobe has a disrupted gene encoding an enzyme selected from the group consisting of the metabolic enzymes of glycolysis except hexokinase, thereby suppressing metabolism from a carbon source to the TCA cycle in the aerobe, the method comprising
culturing the aerobe in a culture medium containing any one of nutrient sources in an aerobic condition.

According to the method (A), in an aerobe, metabolism from a carbon source to the TCA cycle is suppressed, with the result that the carbon source cannot be effectively used for energy production of the aerobe or cannot be sufficiently effectively used. Whereas, the aerobe can more usefully use a carbon source for substance production in the presence of a carbon source. In contrast, the aerobe cannot proliferate or has less proliferation potential in the presence of the carbon source alone, with the result that the aerobe can be aerobically cultured in the presence of a nutrient source. By the mechanism, the aerobe can be proliferated. The carbon source herein is a nutrient as defined in the above and can be a nutrient selected from the group consisting of a substrate and a metabolite for glycolysis in the upstream of a disrupted metabolic enzyme, and a substrate and a metabolite for a metabolic pathway flowing into the upstream glycolysis. The nutrient source is a nutrient as defined in the above and can be a metabolite of glycolysis in the downstream of a disrupted metabolic enzyme, a substrate and a metabolite for a metabolic pathway flowing into downstream glycolysis from the disrupted metabolic enzyme, and a substrate and a metabolite to be consumed in the TCA cycle. Examples of the nutrient source include amino acids except glutamate, acetyl CoA, NADH, FADH₂, GTP, metabolites of the TCA cycle (for example, NADH, malate, succinate, glutamate, α-ketoglutarate, iso-citrate), acetate and protocatechuate.

For example, when phosphofructokinase is disrupted in an aerobe, a carbon source such as glucose and its metabolite (for example, glucose 6-phosphate) used in the upstream of phosphoglycerate kinase is not metabolized in glycolysis in the downstream of phosphofructokinase and the TCA cycle or decreased in metabolic efficiency. Accordingly, the carbon source for a phosphofructokinase disrupted strain can be glucose and glucose 6-phosphate. Examples of the nutrient source for a phosphofructokinase disrupted strain include metabolites of glycolysis in the downstream of phosphofructokinase, amino acids except glutamate, acetyl CoA, NADH, FADH₂, GTP, metabolites of the TCA cycle (for example, NADH, malate, succinate, glutamate, α-ketoglutarate, iso-citrate), acetate and protocatechuate. Glycerol is metabolized into dihydroxyacetone phosphate via glycerol 3-phosphate and used for producing energy in glycolysis and the downstream thereof. Accordingly, glycerol can be a nutrient source for a phosphofructokinase disrupted strain.

If an actinomycete is used as an aerobe, phosphofructokinase A2 and A3 are responsible for metabolism of fructose 6-phosphate into fructose 1,6-bisphosphate (step 3). Accordingly, in an actinomycete, disruption of a metabolic enzyme in step 3 can be achieved by disrupting phosphofructokinase A2 and A3.

In the method (A), an aerobe can be cultured in the presence of a nutrient source. In an embodiment, an aerobe can be cultured in the presence of either one of a metabolite in the downstream of a disrupted metabolic enzyme and a metabolite of the TCA cycle.

In the method (A), an aerobe may be cultured in the presence of a carbon source. If culture is carried out in the presence of a carbon source, the carbon source can be effectively used in the pathway for producing a substance, such as the pentose phosphate pathway.

The aerobe may further have a disrupted transaldolase. If transaldolase is disrupted, the metabolism of sedoheptulose 7-phosphate + glyceraldehyde 3-phosphate into fructose 6-phosphate + erythrose 4-phosphate is inhibited. As a result, the amount of a product of the pentose phosphate pathway flowing into the glycolysis can be reduced.

In the aerobe, phosphoglycerate kinase may be disrupted. For example, in an actinomycete, phosphoglycerate kinase may be disrupted. A phosphoglycerate kinase disrupted strain can be used in the method (A). Examples of a carbon source for a phosphoglycerate kinase disrupted strain include glucose, fructose, glycerol, xylose, arabinose, gluconic acid, trehalose, mannose and ribose. A nutrient source for a phosphoglycerate kinase disrupted strain can be a nutrient selected from the group consisting of 3-phosphoglycerate, 2-phosphoglycerate, phosphoenolpyruvate, pyruvate, amino acids except glutamate, acetyl CoA, NADH, FADH₂, GTP, metabolites of the TCA cycle (for example, NADH, malate, succinate, glutamate, α-ketoglutarate, iso-citrate), acetate and protocatechuate.

In a phosphofructokinase disrupted strain, glycerol is used as a nutrient source. However, in a phosphoglycerate kinase disrupted strain, glycerol is used as not a nutrient source but a carbon source. Accordingly, if the metabolic enzymes disrupted differ, a nutrient serving as a carbon source and a nutrient that can be used as a nutrient source differ. The metabolic enzymes that can be disrupted are those used in step 2 to 10 of glycolysis. In the enzyme-disrupted strain, a carbon source and a nutrient source can be determined by those skilled in the art in consideration of the metabolic pathway.

In the method (A), after an aerobe is cultured in the presence of a nutrient source, the aerobe can be further cultured in the presence of a carbon source (and a nutrient source). In the presence of a nutrient source, an aerobe can produce energy from the nutrient source and actively proliferate. If an aerobe is further cultured in the presence of a carbon source (and a nutrient source) before, during or after proliferation (preferably, during or after proliferation), the aerobe produces a metabolite of the pentose phosphate pathway from the carbon source and a metabolite derived therefrom, a metabolite of glycolysis in the upstream of the disrupted enzyme and a metabolite derived therefrom. In contrast, a metabolic enzyme-disrupted strain utilizes a nutrient source to proliferate and carbon source is subjected to production of a metabolite from the pentose phosphate pathway and metabolite derived therefrom, and production of a metabolite of glycolysis in the upstream of the disrupted enzyme and a metabolite derived therefrom. In this manner, the yield of a metabolite (for example, relative to sugar) can be increased while preventing a decrease in proliferation (see, for example, Figure 13).

In the method (A), after an aerobe is cultured, for example, in the presence of a nutrient source, the aerobe can be cultured in the presence of a carbon source. In this case, proliferation of the aerobe can be promoted in the presence of a nutrient source. Production of a substance by the aerobe can be promoted in the presence of a carbon source. Production of a substance by an aerobe may be carried out in the presence of a nutrient source and a carbon source. If so, production of a substance can be promoted by an aerobe while promoting proliferation of the aerobe.

According to the present invention, there is provided a method for culturing an aerobe, wherein
the aerobe has a disrupted gene encoding a metabolic enzyme of glycolysis selected from the group consisting of the metabolic enzymes of glycolysis except hexokinase, thereby suppressing metabolism from a carbon source to the TCA cycle in the aerobe, the method comprising
culturing the aerobe in a culture medium containing the carbon source under an aerobic condition.

According to the present invention, there is provided a method for producing a metabolite of a pathway selected from a glycolysis, the pentose phosphate pathway and metabolic pathways derived from these, in an aerobe, under an aerobic condition, wherein
the aerobe has a disrupted gene encoding an enzyme selected from the group consisting of the metabolic enzymes of glycolysis except hexokinase, thereby suppressing metabolism from glucose to the TCA cycle in the aerobe, the method comprising
culturing the aerobe in a culture medium containing a carbon source under an aerobic condition.

The metabolite and a metabolite derived therefrom can be recovered from the culture medium. The metabolite and a metabolite derived therefrom can be recovered from the supernatant of a culture medium (for example, secreted metabolite) or from cells cultured (a metabolite accumulated within the cells). Accordingly, in the embodiment, the metabolite or the metabolite derived therefrom may be recovered from a culture in the presence of a carbon source under an aerobic condition after culturing the metabolic enzyme-disrupted strain in the presence of the carbon source under the aerobic condition.

The metabolite to be obtained and a metabolite derived therefrom are mostly industrially useful metabolites. Accordingly, these metabolites are collected after culturing and can be put in useful uses. The "metabolite derived therefrom" herein refers to a metabolite obtained from a metabolic pathway derived from the metabolic pathway specified. For example, a metabolite derived from the pentose phosphate pathway refers to a metabolite of a metabolic pathway derived from the pentose phosphate pathway. A metabolite derived from glycolysis refers to a metabolite of a metabolic pathway derived from glycolysis.

In an embodiment of the present invention, the carbon source may be xylose and the metabolite may be xylulose and/or xylitol. In an embodiment of the present invention, the metabolite may be sedoheptulose. In an embodiment of the present invention, the metabolite may be trehalose.

In an embodiment of the present invention, there is provided a method for producing sedoheptulose in an aerobe under an aerobic condition, wherein
the aerobe has a disrupted gene encoding an enzyme selected from the group consisting of the metabolic enzymes of glycolysis except hexokinase, thereby suppressing metabolism from glucose to the TCA cycle in the aerobe, the method comprising
culturing the aerobe in the culture medium containing a carbon source (for example, glucose) under an aerobic condition.

The culture medium may further contain a nutrient source. The metabolic enzyme to be disrupted, may be, for example, phosphofructokinase; more specifically either one or both of phosphofructokinase A2 and A3. Further, the metabolic enzyme to be disrupted may be transaldolase. According to the present invention, there can be provided a culture containing sedoheptulose obtained by the method of the present invention.

In an embodiment of the present invention, there is provided a method for producing trehalose in an aerobe under an aerobic condition, wherein
the aerobe has a disrupted gene encoding an enzyme selected from the group consisting of the metabolic enzymes of glycolysis except hexokinase, thereby suppressing metabolism from glucose to the TCA cycle in the aerobe, the method comprising
culturing the aerobe in the culture medium containing a carbon source (for example, glucose) in an aerobic condition.

The culture medium may further contain a nutrient source. The metabolic enzyme to be disrupted may be, for example, phosphofructokinase and/or phosphoglycerate kinase, and particularly, phosphoglycerate kinase. According to the present invention, there can be provided a culture containing trehalose obtained by the method of the present invention.

In an embodiment of the present invention, there is provided a method for producing xylulose and/or xylitol in an aerobe under an aerobic condition, wherein
the aerobe has a disrupted gene encoding an enzyme selected from the group consisting of the metabolic enzymes of glycolysis except hexokinase, thereby suppressing metabolism from glucose to the TCA cycle in the aerobe, the method comprising
culturing the aerobe in the culture medium containing a carbon source (for example, xylose) under an aerobic condition.

The culture medium may further contain a nutrient source. The metabolic enzyme to be disrupted may be, for example, phosphoglycerate kinase. According to the present invention, there can be provided a culture containing xylulose and/or xylitol obtained by the method of the present invention.

(B) According to the present invention, there is provided a culture comprising a culture medium used in culturing under an aerobic condition, wherein
the culture medium contains an aerobe, and
the aerobe has a disrupted gene encoding a metabolic enzyme of glycolysis selected from the group consisting of the metabolic enzymes of glycolysis except hexokinase, thereby suppressing metabolism from a carbon source to the TCA cycle in the presence of a carbon source in the aerobe.

The culture (B) can be obtained, for example, by the method (A). More specifically, in the above method (A), a metabolic enzyme-disrupted strain is cultured in the presence of a nutrient source. In this case, the aerobe can proliferate. The metabolic enzyme-disrupted strain not proliferated can produce a substance in the presence of a carbon source. However, to increase the amount of a substance to be produced, the metabolic enzyme-disrupted strain is preferably proliferated by the method (A). For proliferation of a metabolic enzyme-disrupted strain, a carbon source is not required. Accordingly, a carbon source need not be contained in the culture medium. The metabolic enzyme-disrupted strain proliferated can produce a substance in the presence of a carbon source. A nutrient source is not required for producing a substance by a metabolic enzyme-disrupted strain. Accordingly, a nutrient source need not be contained in the culture medium.

The culture (B) may further contain a carbon source or may not contain a carbon source in the culture medium. The carbon source may be a carbon source that has been added to the culture medium before culture. The culture (B) may further contain a nutrient source. The nutrient source may be a nutrient source that has been added to the culture medium before culture.

The culture (B) may contain a carbon source and a nutrient source in the culture medium. The culture (B) may contain neither a carbon source nor a nutrient source in the medium. Such a culture can be obtained, for example, by carrying out culture in accordance with the method (A) until a nutrient source is used up, and thereafter, the culture can be used for culturing in the presence of a carbon source to obtain a substance. The culture (B) may further contain a metabolite of a carbon source and a metabolite derived therefrom (for example, a metabolite selected from the group consisting of sedoheptulose, xylulose, xylitol and trehalose).

In an embodiment of the present invention, the culture (B) may contain sedoheptulose in a concentration of 5 g/L or more, 6 g/L or more, 7 g/L or more, 8 g/L or more, 9 g/L or more, 10 g/L or more, 15 g/L or more, 20 g/L or more, 25 g/L or more, 30 g/L or more, 35 g/L or more or 40 g/L or more.

In an embodiment of the present invention, the culture (B) may contain either one or both of a carbon source and a nutrient source. The type of carbon source and nutrient source can be determined depending on which metabolic enzyme is disrupted in a metabolic enzyme-disrupted strain. For example, the culture (B) may contain glucose, further glycerol, an amino acid and/or succinate.

In an embodiment of the present invention, the culture (B) can contain trehalose in a concentration of 131 mg/L/OD600 or more, 135 mg/L/OD600 or more, 140 mg/L/OD600 or more, 150 mg/L/OD600 or more, 160 mg/L/OD600 or more, 170 mg/L/OD600 or more, 180 mg/L/OD600 or more, 190 mg/L/OD600 or more, 200 mg/L/OD600 or more, 250 mg/L/OD600 or more, 300 mg/L/OD600 or more, 350 mg/L/OD600 or more, 400 mg/L/OD600 or more, 450 mg/L/OD600 or more, 500 mg/L/OD600 or more, 600 mg/L/OD600 or more, 700 mg/L/OD600 or more, 800 mg/L/OD600 or more, 900 mg/L/OD600 or more, 1000 mg/L/OD600 or more, 1100 mg/L/OD600 or more, 1200 mg/L/OD600 or more, 1300 mg/L/OD600 or more, 1400 mg/L/OD600 or more, 1500 mg/L/OD600 or more, 1600 mg/L/OD600 or more, 1700 mg/L/OD600 or more, 1800 mg/L/OD600 or more, 190 mg/L/OD600 or more or 2000 mg/L/OD600 or more.

In an embodiment, the culture (B) can contain xylulose in a concentration of 2 g/L or more, 2.5 g/L or more, 3.0 g/L or more, 3.5 g/L or more, 4.0 g/L or more, 4.5 g/L or more or 5 g/L or more. In an embodiment, the culture (B) can contain xylitol in a concentration of 1.5 g/L or more, 1.6 g/L or more, 1.7 g/L or more, 1.8 g/L or more, 1.9 g/L or more, 2.0 g/L or more, 2.5 g/L or more, 3.0 g/L or more, 3.5 g/L or more, 4.0 g/L or more, 4.5 g/L or more or 5.0 g/L or more.

The culture medium to be used for culturing an aerobe can be appropriately selected by those skilled in the art. The culture medium that can be used may contain a nutrient (for example, nitrogen, phosphorus, sulfur, metal ions and salt) other than a carbon source in a sufficient amount to keep an aerobe alive or for the aerobe to proliferate. In the present invention, a precondition is that a carbon source is consumed as much as possible for producing a substance. Thus, a nutrient source is added to the culture medium in an amount sufficient to produce energy required for proliferation of cells by the TCA cycle from the nutrient source. The culture medium may be a synthetic medium or a semisynthetic medium. The culture medium may contain a basal medium such as TSB medium, LB medium, YT medium (or 2 × YT medium), NZY medium, M9 medium, SOC medium and YPD medium; a buffer; a salt; a pH regulator; an alkaline ion such as potassium ion and sodium ion; an alkaline earth metal ion such as calcium ion; a metal ion such as magnesium ion, iron ion, tin ion, cobalt ion, manganese ion and zinc ion; phosphorus and sulfur. The culture medium may contain a nitrogen source selected from the group consisting of a yeast extract, a hydrolyzed protein, a yeast autolysate, pancreatic digest of casein, soy bean digested with papain, tryptone, phyton-peptone, meat extract, meat peptone and tryptose. The culture medium may contain a salt selected from the group consisting of potassium phosphate, magnesium sulfate, sodium chloride, calcium chloride, borate, copper sulfate, potassium iodide, ferric chloride, manganese (II) sulfate, sodium molybdate and zinc sulfate. The culture medium may contain a sugar such as glucose as a carbon source. The amount of a nutrient source to be added to a culture medium may be specified as that for an aerobe to be cultured to sufficiently proliferate (for example, 1.5 times proliferation, 2 times proliferation, 3 times proliferation, 4 times proliferation, 5 times proliferation, 6 times proliferation, 7 times proliferation, 8 times proliferation, 9 times proliferation, 10 times proliferation) under an aerobic condition. The amount of a nutrient source to be added to a culture medium may be specified as that for an aerobe to sufficiently and increasingly proliferate in the presence of a nutrient source (for example, 1.5 times proliferation, 2 times proliferation, 3 times proliferation, 4 times proliferation, 5 times proliferation, 6 times proliferation, 7 times proliferation, 8 times proliferation, 9 times proliferation, 10 times proliferation) compared to in the absence of the nutrient source. The degree of proliferation can be determined, for example, based on the optical density (OD) as an index, which is measured by an absorption photometer. The optical density can be obtained by the absorbance of light, for example, at a wavelength of 600 nm. Those skilled in the art can appropriately evaluate the degree of proliferation based on OD. The culture medium contains a nutrient sources, and at least one of the concentration of the nutrient sources is, for example, 2.0 mM or more, 2.1 mM or more, 2.2 mM or more, 2.3 mM or more, 2.4 mM or more, 2.5 mM or more, 2.6 mM or more, 2.7 mM or more, 2.8 mM or more, 2.9 mM or more, 3.0 mM or more, 3.1 mM or more, 3.2 mM or more, 3.3 mM or more, 3.4 mM or more, 3.5 mM or more, 3.6 mM or more, 3.7 mM or more, 3.8 mM or more, 3.9 mM or more, 4.0 mM or more, 4.1 mM or more, 4.2 mM or more, 4.3 mM or more, 4.4 mM or more, 4.5 mM or more, 4.6 mM or more, 4.7 mM or more, 4.8 mM or more, 4.9 mM or more, about 4.93 mM, 5 mM or more, 6 mM or more, 7 mM or more, 8 mM or more, 9 mM or more, 10 mM or more, 11 mM or more, 12 mM or more, 13 mM or more, 14 mM or more, 15 mM or more, 16 mM or more, 15 mM or more, 18 mM or more, about 18.5 mM, 19 mM or more, 20 mM or more, 30 mM or more, about 30 mM or more, 40 mM or more, 48 mM or more, 50 mM or more, about 56 mM or more, 60 mM or more, about 61 mM or more, 70 mM or more, 80 mM or more, 90 mM or more, about 91 mM or more, 100 mM or more, 153 mM or more, 200 mM or more, 300 mM or more, 400 mM or more, 500 mM or more, about 543 mM or more or 600 mM or more. The culture medium contains, for example, citrate. The concentration of citrate is sufficient for cells to proliferate and, for example, beyond 2.0 mM. The culture medium contains, for example, succinate. The concentration of succinate is sufficient for cells to proliferate and, for example, beyond 2.0 mM. The culture medium contains, for example, alanine, which is an amino acid. The concentration of alanine is sufficient for cells to proliferate and, for example, beyond 2.0 mM. The culture medium contains, for example, glutamate. The concentration of glutamate is sufficient for cells to proliferate and, for example, beyond 2.0 mM. The culture medium contains, for example, protocatechuate. The concentration of protocatechuate is sufficient for cells to proliferate and, for example, beyond 2.0 mM. The culture medium contains, for example, a nutrient source, and at least of the concentration of the nutrient sources may be 0.2 g/L or more, 0.3 g/L or more, 0.4 g/L or more, 0.5 g/L or more, 0.6 g/L or more, 0.7 g/L or more, 0.8 g/L or more, 0.9 g/L or more, 1 g/L or more, 1.5 g/L or more, 2 g/L or more, 3 g/L or more, 4 g/L or more, 5 g/L or more, 6 g/L or more, 7 g/L or more, 8 g/L or more, 9 g/L or more, 10 g/L or more, 20 g/L or more, 30 g/L or more, 40 g/L or more, 50 g/L or more, 60 g/L or more, 70 g/L or more, 80 g/L or more, 90 g/L or more or 100 g/L or more. The nutrient source to be added to the culture medium is determined so as to fall within a suitable concentration range for cell proliferation. The amount of a carbon source to be added to a culture medium can be an effective amount for substance production. The amount of a carbon source to be added to a culture medium can be appropriately determined by those skilled in the art in consideration of the amount of a substance to be produced and the production efficiency. The type of carbon source to be added to a culture medium can be appropriately determined by those skilled in the art in consideration of the amount of a substance to be produced and the production efficiency.

Culture conditions suitable for culturing an aerobe can be appropriately determined by those skilled in the art. Particularly, culture conditions for culturing an aerobe (an aerobe suitable for substance production and an aerobe suitable for culture) for use in substance production are well known to those skilled in the art. In the present invention, an aerobe suitable for substance production can be used as an aerobe. Culturing time can be appropriately determined by those skilled in the art in light of the proliferation state of an aerobe and the production amount of a substance. Culturing time, although it is not particularly limited, is, for example, 12 hours to 500 hours, 24 hours to 400 hours, 24 hours to 300 hours, 24 hours to 200 hours, 100 hours to 300 hours, 100 hours to 200 hours or 24 hours to 100 hours. During culture, a carbon source and/or a nutrient source can be added to a culture medium so as for the carbon source and/or nutrient source not to deplete.

### Examples

### (Example 1)

### 1. Production of sedoheptulose by Streptomyces

The present inventors prepared a sedoheptulose-producing strain of *Streptomyces lividans* and examined the cell turbidity of a culture solution and amount of sedoheptulose in the culture solution.

### 1-1. Disruption of metabolic gene

### 1-1-1. Disruption of transaldolase gene of

### Streptomyces lividans

A transaldolase gene (SLI_2249) of *Streptomyces lividans* 1326 strain (NITE deposit number: NBRC 15675) was disrupted by homologous recombination. Transformation of *Streptomyces lividans* was carried out in accordance with a method known in the technical field.

### 1-1-2. Disruption of phosphofructokinase A3 gene of

### Streptomyces lividans

The transaldolase gene (SLI_2249)-disrupted strain of *Streptomyces lividans* 1326 was used as a host and phosphofructokinase A3 gene (SLI_1493) was disrupted by homologous recombination. Transformation of *Streptomyces lividans* was carried out in accordance with a method known in the technical field.

### 1-1-3. Disruption of phosphofructokinase A2 gene of

### Streptomyces lividans

The transaldolase gene (SLI_2249)- and phosphofructokinase A3 gene (SLI_1493)-disrupted strain of *Streptomyces lividans* 1326 strain was used as a host and phosphofructokinase A2 gene (SLI_5695) was disrupted by homologous recombination. Transformation of *Streptomyces lividans* was carried out in accordance with a method known in the technical field.

### 1-2. Preculture of Streptomyces lividans

To 10 mL of TSB medium (see, the following Table 1), a glycerol stock of *Streptomyces lividans* 1326 strain, *Streptomyces lividans* 1326ΔSLI_2249 strain, *Streptomyces lividans* 1326ΔSLI_2249ΔSLI_1493 strain or *Streptomyces lividans* 1326ΔSLI_2249ΔSLI_1493ΔSLI_5695 strain was added. Shaking culture of each of these actinomycetes was carried out at 28°C at 160 rpm for 48 to 72 hours.

### 1-3. Main culture of Streptomyces lividans

### 1-3-1. Culture of Streptomyces lividans in the presence of glucose

The preculture solution (0.1% in amount) was added to 50 mL of TSB medium in a 500 mL-flask with a baffle (see, the following Table 1). Note that glucose was further added to the TSB medium at the start of culture such that the initial concentration of glucose became 80 g/L. Shaking culture was carried out at 28°C at 160 rpm for 10 to 12 days, while adding glucose to the TSB medium so as for glucose not to deplete.

### 1-3-2. Streptomyces lividans in the presence of glycerol

The preculture solution (0.1% in amount) was added to 50 mL of TSB medium in a 500 mL-flask with a baffle. Note that further glycerol was added to the TSB medium at the start of culture such that the initial concentration of glycerol became 50 g/L (about 543 mM). Shaking culture was carried out at 28°C at 160 rpm for 10 to 12 days while adding glucose before glycerol depleted and thereafter adding glucose so as for glucose not to deplete.

**[Table 1]**

| TSB medium | |
|---|---|
| pancreatic digest of casein | 17 g (1.7%) |
| Soy bean digested with papain | 3 g (0.3%) |
| Glucose | 25 g (0.25%) |
| NaCl | 5 g (0.5%) |
| K₂HPO₄ | 2.5 g (0.25%) |
| Total | 1000 mL |

### 1-4. Measurement of sedoheptulose

A culture solution (1 mL) was collected during the main culture at predetermined time points and the turbidity of the culture solutions was measured at 600 nm. The culture solutions taken were each centrifuged at 14,000 rpm for 20 minutes to obtain culture solution samples. The amounts of sedoheptulose produced in the culture solution samples were measured by HPLC under the conditions shown in the following table.

**[Table 2]**

| Column: | Aminex HPX-87C (9 µm 7.8 φ mm × 300 mm) (manufactured by BIO-RAD) |
|---|---|
| Solvent: | H₂O |
| Detector: | RID |
| Reference sample: | Sedoheptulose manufactured by SIGMA |
| Flow rate: | 0.6 mL/minute |
| Column temperature: | 85°C |
| Retention time: | Sedoheptulose: around 11.5 minutes |

### 1-5. Results

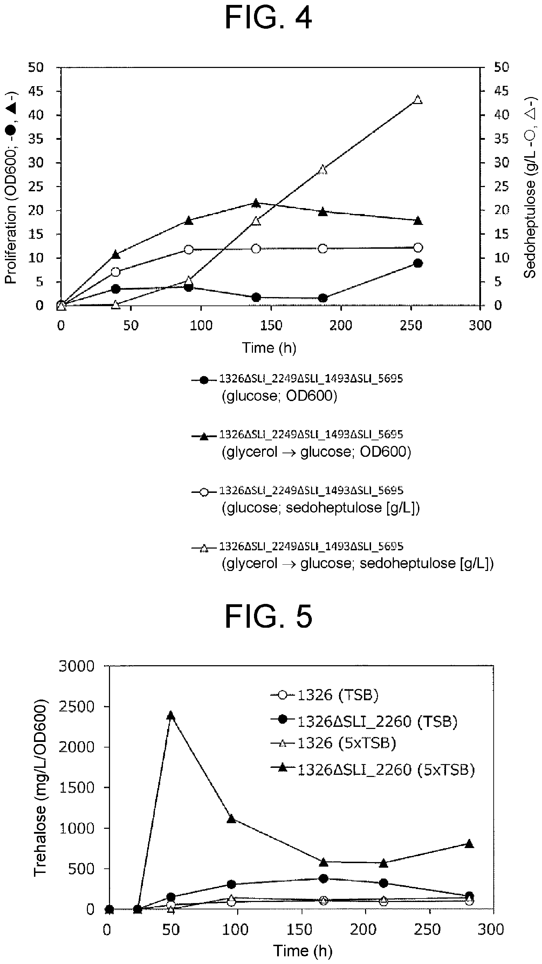

The results of *Streptomyces lividans* 1326 strain are shown in Figure 1; the results of *Streptomyces lividans* 1326ΔSLI_2249 strain in Figure 2; the results of *Streptomyces lividans* 1326ΔSLI_2249ΔSLI_1493 strain in Figure 3; and the results of *Streptomyces lividans* 1326ΔSLI_2249ΔSLI_1493ΔSLI_5695 strain in Figure 4.

In *Streptomyces lividans* 1326 strain, no sedoheptulose production was confirmed in a culture in the presence of glucose or a culture in the presence of glycerol + glucose, for 12 days. Whereas, in the case of *Streptomyces lividans* 1326ΔSLI_2249 strain cultured in the presence of glucose alone, the OD600 value at a culture time of 281 hours was 31.6; the amount of sedoheptulose was 1.6 g/L; and the yield relative to sugar was 1.3%. In contrast, in the case of a culture in the presence of glycerol and glucose, the OD600 value at a culture time of 255 hours was 37.6; the amount of sedoheptulose was 2.9 g/L; and the yield relative to sugar was 2.5%. In the case where *Streptomyces lividans* 1326ΔSLI_2249ΔSLI_1493 strain was cultured in the presence of glucose alone, the OD600 value at a culture time of 281 hours was 37.2; the amount of sedoheptulose was 2.6 g/L; and the yield relative to sugar was 1.7%. In contrast, in the case of a culture in the presence of glycerol and glucose, the OD600 value at a culture time of 255 hours was 40.8; the amount of sedoheptulose was 4.8 g/L; and the yield relative to sugar was 3.1%. In the case where *Streptomyces lividans* 1326ΔSLI_2249ΔSLI_1493ΔSLI_5695 strain was cultured in the presence of glucose alone, the OD600 value at a culture time of 255 hours was 8.9; the amount of sedoheptulose was 12.2 g/L and the yield relative to sugar was 21.7%. In contrast, in the case of a culture in the presence of glycerol and glucose, the OD600 value at a culture time of 255 hours was 17.9; the amount of sedoheptulose was 43.3 g/L; and the yield relative to sugar was 41.3%.

In the cases where 1326ΔSLI_2249 strain, 1326ΔSLI_2249ΔSLI_1493 strain and 1326ΔSLI_2249ΔSLI_1493ΔSLI_5695 strain were each cultured in the presence of glycerol + glucose, compared to cases where they were each cultured in the presence of glucose, OD600 values, amounts of sedoheptulose and the yields of sedoheptulose relative to sugar were all improved.

### (Example 2)

### 2. Production of trehalose by Streptomyces

The present inventors prepared a trehalose-producing strain of *Streptomyces lividans* and examined trehalose production per cell.

### 2-1. Disruption of metabolic gene

### 2-1-1. Disruption of phosphoglycerate kinase gene in Streptomyces lividans

Phosphoglycerate kinase gene (SLI_2260) of *Streptomyces lividans* 1326 strain (NITE deposit number: NBRC 15675) was disrupted by homologous recombination. Transformation of *Streptomyces lividans* was carried out in accordance with a method known in the technical field.

### 2-2. Preculture of Streptomyces lividans

To 10 mL of TSB medium, a glycerol stock of *Streptomyces lividans* 1326 strain or *Streptomyces lividans* 1326ΔSLI_2260 strain prepared in Section 2-1 was added. Shaking culture of each of these actinomycetes was carried out at 28°C at 160 rpm for 72 to 96 hours.

### 2-3. Main culture of Streptomyces lividans

### 2-3-1. Culture of Streptomyces lividans in TSB in the presence of glucose

The preculture solution (0.1% in amount) was added to 50 mL of TSB medium in a 500 mL-flask with a baffle. Note that, glucose was further added to the TSB medium at the start of culture such that the initial concentration of glucose became 50 g/L. Shaking culture was carried out at 28°C at 160 rpm for 10 to 12 days while adding glucose so as for glucose not to deplete.

### 2-3-2. Culture of Streptomyces lividans in 5× TSB in the presence of glucose

The preculture solution (0.1% in amount) was added to 50 mL of TSB medium (concentrated 5-fold) in 500 mL in a flask with a baffle. Note that glucose was further added to the medium at the start of culture such that an initial concentration of glucose became 50 g/L. Shaking culture was carried out at 28°C at 160 rpm for 10 to 12 days while adding glucose so as for glucose not to deplete.

### 2-4. Measurement of trehalose and calculation of trehalose production per cell

A culture solution (1 mL) was collected during the main culture at predetermined time points and turbidity of the culture solutions was measured at 600 nm. The culture solutions taken were each centrifuged at 14,000 rpm for 20 minutes to obtain culture solution samples. The amounts of trehalose produced in the culture solution samples were measured by HPLC under the conditions shown in the following table.

The amount of trehalose produced was divided by the cell turbidity to obtain a trehalose production per unit cell.

**[Table 3]**

| Column: | Aminex HPX-87C (9 µm 7.8 φ mm × 300 mm) (manufactured by BIO-RAD) |
|---|---|
| Solvent: | H₂O |
| Detector: | RID |
| Reference sample: | Trehalose manufactured by Nacalai Tesque Inc. |
| Flow rate: | 0.6 mL/minute |
| Column temperature: | 85°C |
| Retention time: | Trehalose: around 8.5 minutes |

### 2-5. Results

The production amounts of *Streptomyces lividans* 1326 strain and *Streptomyces lividans* 1326ΔSLI_2260 strain per unit cell are shown in Figure 5.

The trehalose production of *Streptomyces lividans* 1326 strain cultured in TSB medium for 281 hours was 98 mg/L/OD600. In contrast, when the strain was cultured in 5× TSB medium, the trehalose production thereof increased up to 130 mg/L/OD600. Whereas, the trehalose production of *Streptomyces lividans* 1326ΔSLI_2260 strain cultured in TSB medium was 226 mg/L/OD600. In contrast, when the strain was cultured in 5× TSB medium, the production thereof significantly increased up to 808 mg/L/OD600.

### (Example 3)

### 3-1. Proliferative study of Streptomyces lividans 1326ΔSLI_2260 strain

When *Streptomyces lividans* 1326ΔSLI_2260 strain, in which its metabolism is suppressed, was cultured, the effect of the added substance in the downstream of the disrupted metabolic enzymewas examined and compared to those of the cases where glucose and glycerol were each individually added as carbon sources.

### 3-2. Preculture of Streptomyces lividans

To 10 mL of TSB medium, a glycerol stock of *Streptomyces lividans* 1326 strain or *Streptomyces lividans* 1326ΔSLI_2260 strain prepared in Section 2-1 was added. Shaking culture of each of these actinomycetes was carried out at 28°C at 160 rpm for 72 to 96 hours.

### 3-3. Main culture of Streptomyces lividans

### 3-3-1. Culture of Streptomyces lividans in TSB in the presence of glucose

The preculture solution (0.1% in amount) was added to 50 mL of TSB medium in a 500 mL-flask with a baffle. Note that glucose was further added to the TSB medium at the start of culture such that the initial concentration of glucose became 50 g/L. Shaking culture was carried out at 28°C at 160 rpm for 7 days while adding glucose so as for glucose not to deplete.

### 3-3-2. Culture of Streptomyces lividans in TSB in the presence of glucose and protocatechuate

The preculture solution (0.1% in amount) was added to 50 mL of TSB medium in a 500 mL-flask with a baffle. Note that, protocatechuate serving as a nutrient source was added to the TSB medium at the start of culture such that the initial concentration of protocatechuate became 0.76 g/L (about 4.93 mM). On day 1, 2 and 3 after the start of culture, 5 mL of a 7.6 g/L protocatechuate solution was added. On day 4, 6 mL of a 500 g/L glucose solution was added. Thereafter, shaking culture was carried out at 28°C at 160 rpm for 7 days while adding glucose so as for glucose not to deplete.

### 3-3-3. Culture of Streptomyces lividans in TSB in the presence of glucose and alanine

The preculture solution (0.1% in amount) was added to 50 mL of TSB medium in a 500 mL-flask with a baffle. Note that glucose and alanine were added to the TSB medium at the start of culture such that the initial concentration of glucose became 50 g/L and the initial concentration of alanine serving as a nutrient source became 5 g/L (about 56 mM). On day 2, 3 and 4 after the start of culture, 2.5 mL of a 100 g/L alanine solution was added. Shaking culture was carried out at 28°C at 160 rpm for 7 days while adding glucose so as for glucose not to deplete. The consumption of alanine until immediately before alanine was added on day 3 was 0.273 g. From this, it was found that the content of at least 0.273 g (about 61 mM) of alanine in the initial medium is sufficient for proliferation.

### 3-3-4. Culture of Streptomyces lividans in TSB in the presence of glucose and serine

The preculture solution (0.1% in amount) was added to 50 mL of TSB medium in a 500 mL-flask with a baffle. Note that glucose and serine were added to the TSB medium at the start of culture such that the initial concentration of glucose became 50 g/L and the initial concentration of serine serving as a nutrient source became 5 g/L (about 48 mM). On day 2, 3 and 4 after the start of culture, 2.5 mL of a 100 g/L serine solution was added. Shaking culture was carried out at 28°C at 160 rpm for 7 days while adding glucose so as for glucose not to deplete.

### 3-3-5. Culture of Streptomyces lividans in TSB in the presence of glucose and monosodium glutamate

The preculture solution (0.1% in amount) was added to 50 mL of TSB medium in a 500 mL-flask with a baffle. Note that, glucose and monosodium glutamate were added to the TSB medium at the start of culture such that the initial concentration of glucose became 50 g/L and the initial concentration of monosodium glutamate serving as a nutrient source became 5 g/L (about 30 mM). On day 2, 3 and 4 after the start of culture, 2.5 mL of a 100 g/L monosodium glutamate solution was further added. Shaking culture was carried out at 28°C at 160 rpm for 7 days while adding glucose so as for glucose not to deplete.

### 3-3-6. Culture of Streptomyces lividans in TSB in the presence of glycerol

The preculture solution (0.1% in amount) was added to 50 mL of TSB medium in a 500 mL-flask with a baffle. Note that, glycerol was added to the TSB medium at the start of culture such that the initial concentration of glycerol became 50 g/L. Shaking culture was carried out at 28°C at 160 rpm for 10 days while adding glycerol so as for glycerol not to deplete.

### 3-3-7. Culture of Streptomyces lividans in TSB in the presence of glycerol and alanine

The preculture solution (0.1% in amount) was added to 50 mL of TSB medium in a 500 mL-flask with a baffle. Note that, glycerol and alanine were added to the TSB medium at the start of culture such that the initial concentration of glycerol became 50 g/L and the initial concentration of alanine became 5 g/L. On day 1 and 2 after the start of culture, 5 mL of a 100 g/L alanine solution was added. Shaking culture was carried out at 28°C at 160 rpm for 10 days while adding glycerol so as for glycerol not to deplete.

### 3-3-8. Culture of Streptomyces lividans in TSB in the presence of glycerol and disodium succinate

The preculture solution (0.1% in amount) was added to 50 mL of TSB medium in a 500 mL-flask with a baffle. Note that glycerol and disodium succinate hexahydrate were added to the TSB medium at the start of culture such that the initial concentration of glycerol became 50 g/L and the initial concentration of disodium succinate hexahydrate serving as a nutrient source became 5 g/L (about 18.5 mM). On day 1 and 2 after the start of culture, 5 mL of a 100 g/L disodium succinate solution was added. Shaking culture was carried out at 28°C at 160 rpm for 10 days while adding glycerol so as for glycerol not to deplete.

### 3-4. Measurement of cell turbidity

A culture solution (1 mL) was collected during the main culture at predetermined time points and turbidity was measured at 600 nm.

### 3-5. Results

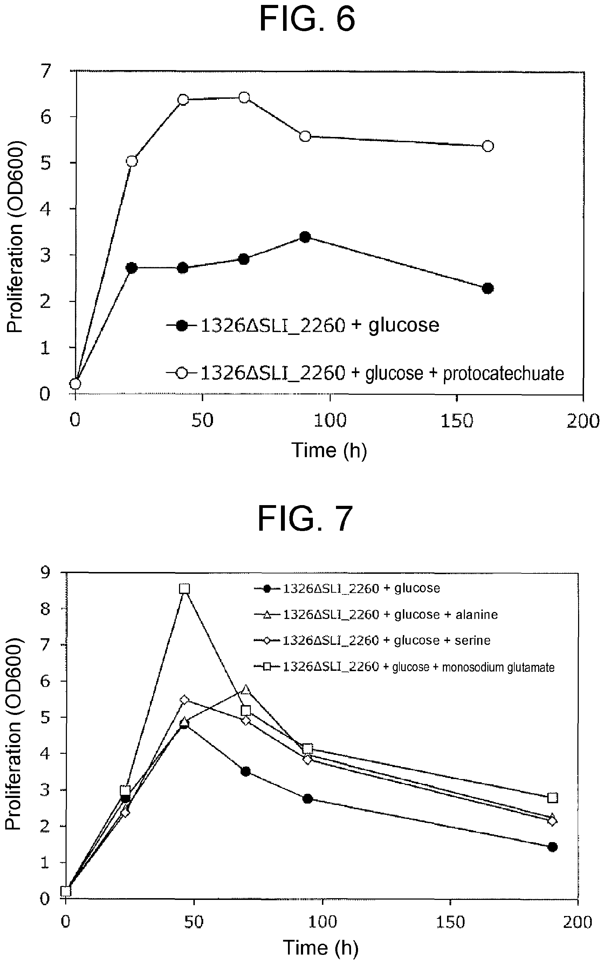

As shown in Figure 6 and Figure 7, in the cases where *Streptomyces lividans* 1326ΔSLI_2260 strain was cultured in the presence of protocatechuate, alanine, serine and monosodium glutamate serving as a nutrient source, the cell turbidity increased, in comparison with the case where the strain was cultured in the presence of glucose alone used as a nutrient source.

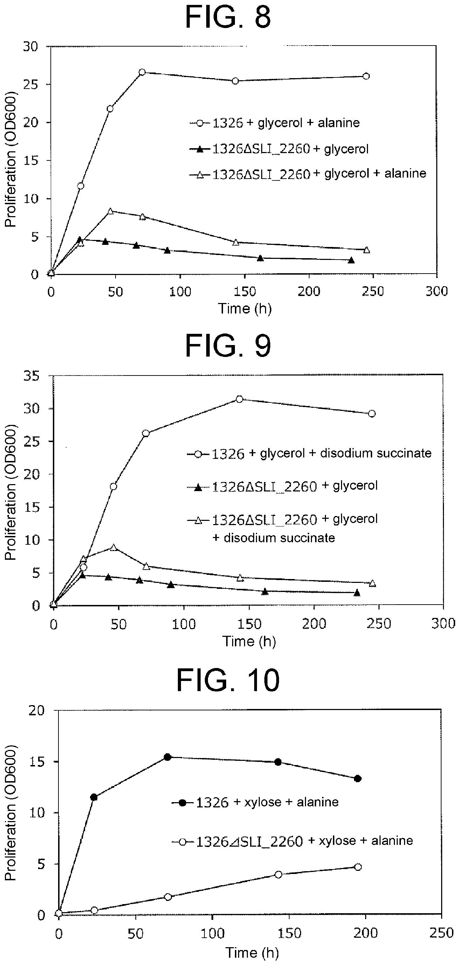

As shown in Figure 8 and Figure 9, in the cases where *Streptomyces lividans* 1326ΔSLI_2260 strain was cultured in the presence of alanine and disodium succinate added as a nutrient source, the cell turbidity increased, in comparison with the case where the strain was cultured in the presence of glycerol alone used as a nutrient source.

### (Example 4)

### 4-1. Production of substance using Streptomyces lividans

*Streptomyces lividans* 1326 and *Streptomyces lividans* 1326ΔSLI_2260 strain, which was suppressed in metabolism, were cultured in the presence of xylose as a carbon source. To the culture mediums, alanine was added. Proliferation and metabolites in these cases were compared.

### 4-2. Preculture of Streptomyces lividans

To 10 mL of TSB medium, a glycerol stock of *Streptomyces lividans* 1326 strain or *Streptomyces lividans* 1326ΔSLI_2260 strain prepared in Section 2-1 was added. Shaking culture of each of these actinomycetes was carried out at 28°C at 160 rpm for 72 to 96 hours.

### 4-3. Main culture of Streptomyces lividans

### 4-3-1 Culture of Streptomyces lividans in TSB in the presence of xylose and alanine

The preculture solution (0.1% in amount) was added to 50 mL of TSB medium in a 500 mL-flask with a baffle. Note that, xylose and alanine were added to the TSB medium at the start of culture such that the initial concentration of xylose became 50 g/L and the initial concentration of alanine serving as a nutrient source became 5 g/L (about 56 mM). On day 2 after the start of culture, 5 mL of a 100 g/L alanine solution was added and 5 mL of 1 M phosphate buffer (pH6.0) was added for pH adjustment. Shaking culture was carried out at 28°C at 160 rpm for 10 days while adding xylose so as for xylose not to deplete.

### 4-4. Measurement of cell turbidity

A culture solution (1 mL) was collected during the main culture at predetermined time points and turbidity of the culture solutions was measured at 600 nm.

### 4-5. Measurement of metabolite

A culture solution (1 mL) was collected during the main culture at predetermined time points and turbidity of the culture solutions was measured at 600 nm. The culture solutions taken were each centrifuged at 14,000 rpm for 20 minutes to obtain culture solution samples. The amounts of a metabolite produced in the culture solution samples were measured by HPLC under the conditions shown in the following table.

**[Table 4]**

| Column: | Aminex HPX-87C (9 µm 7.8 φ mm × 300 mm) (manufactured by BIO-RAD) |
|---|---|
| Solvent: | H₂O |
| Detector: | RID |
| Flow rate: | 0.6 mL/minute |
| Column temperature: | 85°C |
| Retention time: | Xylulose: around 13.4 minutes |
| | Xylitol: around 20.4 minutes |

### 4-6. Results

Cell proliferation is shown in Figure 10. Not only *Streptomyces lividans* 1326 strain but also *Streptomyces lividans* 1326ΔSLI_2260 strain satisfactorily proliferated in the culture in the presence of xylose + alanine. The results of the amount of xylulose (of the metabolites obtained by culture) are shown in Figure 11 and the results of the amount of xylitol are shown in Figure 12. The production amounts of xylulose and xylitol both increased in *Streptomyces lividans* 1326ΔSLI_2260 strain, compared to *Streptomyces lividans* 1326 strain.

## Claims

1. A culture comprising a culture medium used in culturing under an aerobic condition, wherein
the culture medium contains an aerobe and a nutrient source, wherein the nutrient source contains a nutrient source in an amount sufficient for the aerobe to proliferate under the aerobic environment,
the aerobe has a disrupted gene encoding a metabolic enzyme of glycolysis selected from the group consisting of the metabolic enzymes of glycolysis except hexokinase, thereby suppressing metabolism from a carbon source to the TCA cycle in the aerobe, and the carbon source is a nutrient selected from the group consisting of a substrate and a metabolite for glycolysis in the upstream of the metabolic enzyme disrupted, and a substrate and a metabolite for a metabolic pathway flowing into the upstream glycolysis, and
the aerobe allows for metabolism from the nutrient source to the TCA cycle in the presence of the nutrient source for the TCA cycle, and the nutrient source contains a nutrient selected from the group consisting of a metabolite of glycolysis in the downstream of the metabolic enzyme disrupted, a substrate and a metabolite for the metabolic pathway flowing into the downstream glycolysis, a metabolite of the TCA cycle, and a substrate and a metabolite for a metabolic pathway flowing into the TCA cycle.

2. The culture according to claim 1, wherein the culture medium further contains the carbon source.

3. The culture according to claim 1 or 2, wherein at least one of the nutrient sources has a concentration of 2.0 mM or more.

4. The culture according to claim 3, wherein at least one of the nutrient sources has a concentration of 3.0 mM or more.

5. The culture according to any one of claims 1 to 4, wherein the aerobe further has a disrupted gene encoding transaldolase.

6. The culture according to any one of claims 1 to 5, wherein the disrupted gene encoding an enzyme of glycolysis is a gene encoding phosphofructokinase.

7. The culture according to claim 6, wherein the disrupted gene encoding an enzyme of glycolysis is genes encoding phosphofructokinase A2 and A3.

8. The culture according to claim 6 or 7, wherein the nutrient source is at least one molecule selected from the group consisting of glycerol, fructose 1,6-bisphosphate, glyceraldehyde 3-phosphate, dihydroxyacetone phosphate, 1,3-bisphosphoglycerate, 3-phosphoglycerate, 2-phosphoglycerate, phosphoenolpyruvate, pyruvate, acetate, NADH, malate, succinate, glutamate, α-ketoglutarate, iso-citrate, protocatechuate and amino acids except glutamate.

9. The culture according to any one of claims 1 to 4, wherein the disrupted gene encoding an enzyme of glycolysis is a phosphoglycerate kinase gene.

10. The culture according to claim 9, wherein the nutrient source is at least one molecule selected from the group consisting of 3-phosphoglycerate, 2-phosphoglycerate, phosphoenolpyruvate, pyruvate, acetate, NADH, malate, succinate, glutamate, α-ketoglutarate, iso-citrate, protocatechuate and amino acids except glutamate.

11. A method for obtaining a metabolite of a metabolic pathway selected from the group consisting of glycolysis, the pentose phosphate pathway and metabolic pathways derived from these, the method comprising providing the culture according to any one of claims 1 to 10 and obtaining any one of the metabolites from the culture provided.

12. A method for culturing an aerobe, wherein
the aerobe has a disrupted gene encoding a metabolic enzyme of glycolysis selected from the group consisting of the metabolic enzymes of glycolysis except hexokinase, thereby suppressing metabolism from a carbon source to the TCA cycle in the aerobe, and the carbon source is a nutrient selected from the group consisting of a substrate and a metabolite for glycolysis in the upstream of the metabolic enzyme disrupted, and a substrate and a metabolite for a metabolic pathway flowing into the upstream glycolysis, the method comprising
culturing the aerobe in a culture medium containing the carbon source and a nutrient source under an aerobic condition, wherein the nutrient source contains a nutrient source in an amount sufficient for the aerobe to proliferate under an aerobic environment.

13. The method according to claim 12, wherein the aerobe further has a disrupted gene encoding transaldolase.

14. The method according to claim 12 or 13, wherein the disrupted gene encoding an enzyme of glycolysis is a gene encoding phosphofructokinase.

15. The method according to claim 14, wherein the disrupted gene encoding an enzyme of glycolysis is genes encoding phosphofructokinase A2 and A3.

16. The method according to claim 14 or 15, wherein the culture medium further contains at least one molecule selected from the group consisting of glycerol, acetate, fructose 1,6-bisphosphate, glyceraldehyde 3-phosphate, dihydroxyacetone phosphate, 1,3-bisphosphoglycerate, 3-phosphoglycerate, 2-phosphoglycerate, phosphoenolpyruvate, pyruvate, NADH, malate, succinate, glutamate, α-ketoglutarate, iso-citrate, protocatechuate and amino acids except glutamate.

17. The method according to claim 12 or 13, wherein the disrupted gene encoding an enzyme of glycolysis is a phosphoglycerate kinase gene.

18. The method according to claim 17, wherein the culture medium further contains at least one molecule selected from the group consisting of 3-phosphoglycerate, 2-phosphoglycerate, phosphoenolpyruvate, pyruvate, acetate, NADH, malate, succinate, glutamate, α-ketoglutarate, iso-citrate, protocatechuate and amino acids except glutamate.

19. A method for producing a metabolite of a metabolic pathway selected from the group consisting of glycolysis, the pentose phosphate pathway and metabolic pathways derived from these, in an aerobe, under an aerobic condition, wherein
the aerobe has a disrupted gene encoding a metabolic enzyme of glycolysis selected from the group consisting of metabolic enzymes of glycolysis except hexokinase, thereby suppressing metabolism from a carbon source to the TCA cycle in the aerobe, and the carbon source is a nutrient selected from the group consisting of a substrate and a metabolite for glycolysis in the upstream of the metabolic enzyme disrupted, and a substrate and a metabolite for a metabolic pathway flowing into the upstream glycolysis, the method comprising
culturing the aerobe in a culture medium containing the carbon source under an aerobic condition, wherein the culture medium further contains a nutrient source, or a nutrient source and/or the carbon source may be added to the culture medium in the middle of culture, and the nutrient source contains a nutrient source in an amount sufficient for the aerobe to proliferate under an aerobic condition, and
the nutrient source contains a nutrient selected from the group consisting of a metabolite of glycolysis in the downstream of the metabolic enzyme disrupted, a substrate and a metabolite for a metabolic pathway flowing into the downstream glycolysis, a metabolite of the TCA cycle, and a substrate and a metabolite for a metabolic pathway flowing into the TCA cycle.

20. The method according to claim 19, wherein the aerobe further has a disrupted gene encoding transaldolase.

21. The method according to claim 19 or 20, wherein the disrupted gene encoding an enzyme of glycolysis is a gene encoding phosphofructokinase.

22. The method according to claim 21, wherein the disrupted gene encoding an enzyme of glycolysis is genes encoding phosphofructokinase A2 and A3.

23. The method according to claim 21 or 22, wherein the culture medium further contains at least one molecule selected from the group consisting of glycerol, acetate, fructose 1,6-bisphosphate, glyceraldehyde 3-phosphate, dihydroxyacetone phosphate, 1,3-bisphosphoglycerate, 3-phosphoglycerate, 2-phosphoglycerate, phosphoenolpyruvate, pyruvate, NADH, malate, succinate, glutamate, α-ketoglutarate, iso-citrate, protocatechuate and amino acids except glutamate.

24. The method according to claim 19 or 20, wherein the disrupted gene encoding an enzyme of glycolysis is a phosphoglycerate kinase gene.

25. The method according to claim 24, wherein the culture medium further contains at least one molecule selected from the group consisting of 3-phosphoglycerate, 2-phosphoglycerate, phosphoenolpyruvate, pyruvate, acetate, NADH, malate, succinate, glutamate, α-ketoglutarate, iso-citrate, protocatechuate and amino acids except glutamate.

26. A method for proliferating an aerobe under an aerobic condition, the method comprising culturing the aerobe in the presence of a nutrient source under an aerobic condition, wherein
the aerobe has a disrupted gene encoding a metabolic enzyme of glycolysis selected from the group consisting of the metabolic enzymes of glycolysis except hexokinase, thereby suppressing metabolism from a carbon source to the TCA cycle in the aerobe, and the carbon source is a nutrient selected from the group consisting of a substrate and a metabolite for glycolysis in the upstream of the metabolic enzyme disrupted, and a substrate and a metabolite for a metabolic pathway flowing into the upstream glycolysis,
the nutrient source contains a nutrient source in an amount sufficient for the aerobe to proliferate under the aerobic environment, and
the nutrient source contains a nutrient selected from the group consisting of a metabolite of glycolysis in the downstream of the metabolic enzyme disrupted, a substrate and a metabolite for a metabolic pathway flowing into the downstream glycolysis, a metabolite of the TCA cycle, and a substrate and a metabolite for a metabolic pathway flowing into the TCA cycle.

27. An actinomycete having disrupted genes encoding phosphofructokinase A2 and A3.

28. The actinomycete according to claim 27, wherein the actinomycete further has a disrupted gene encoding transaldolase.

29. An actinomycete having a disrupted gene encoding phosphoglycerate kinase.
